# EUROPEAN PATENT SPECIFICATION

(11) **EP 4 630 114 B1**
(45) Date of publication and mention of the grant of the patent: **25.11.2026**
(21) Application number: 23818436.0
(22) Date of filing: 06.12.2023
(51) Int. Cl.: A61P 3/10, A61P 29/00, C07D 213/85, C07D 401/04, C07D 487/04, C07F 9/40

(54) **NEW CYANOPYRIDINE KHK INHIBITOR COMPOUNDS**
NEUE CYANOPYRIDIN-KHK-HEMMERVERBINDUNGEN
NOUVEAUX COMPOSÉS INHIBITEURS DE CYANOPYRIDINE KHK

(30) Priority: 08.12.2022 EP 22212311
(43) Date of publication of application: 15.10.2025
(73) Proprietor: Boehringer Ingelheim International GmbH, 55216 Ingelheim am Rhein (DE)
(72) Inventor: KLEY, Joerg, 55216 Ingelheim am Rhein (DE); GOTTSCHLING, Dirk, 55216 Ingelheim am Rhein (DE); HEINE, Niklas, 55216 Ingelheim am Rhein (DE); NOSSE, Bernd, 55216 Ingelheim am Rhein (DE); PAUTSCH, Alexander, 55216 Ingelheim am Rhein (DE); WEBER, Alexander, 55216 Ingelheim am Rhein (DE)
(74) Representative: Boehringer Global Patents
(86) International application number: PCT/EP2023/084413
(87) International publication number: WO 2024/121183

(56) References cited:
- WO-A1-2006/105023
- WO-A1-2020/051058
- FUTATSUGI KENTARO ET AL: "Discovery of PF-06835919: A Potent Inhibitor of Ketohexokinase (KHK) for the Treatment of Metabolic Disorders Driven by the Overconsumption of Fructose", JOURNAL OF MEDICINAL CHEMISTRY, vol. 63, no. 22, 25 November 2020 (2020-11-25), US, pages 13546 - 13560, XP055923083, ISSN: 0022-2623, DOI: 10.1021/acs.jmedchem.0c00944
- KIM HUARD ET AL: "Discovery of Fragment-Derived Small Molecules for in Vivo Inhibition of Ketohexokinase (KHK)", JOURNAL OF MEDICINAL CHEMISTRY, vol. 60, no. 18, 11 September 2017 (2017-09-11), US, pages 7835 - 7849, XP055643458, ISSN: 0022-2623, DOI: 10.1021/acs.jmedchem.7b00947

## Description

### Field of the invention

The present invention relates to new Cyanopyridine compounds and derivatives of formula **(I)** inhibiting ketohexokinase (KHK) 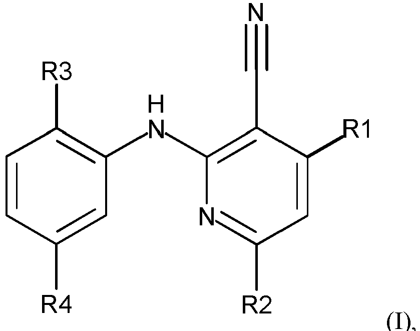 wherein the groups **R1** to **R4** have the meanings given in the claims and specification, their use as inhibitors of KHK, pharmaceutical compositions which contain compounds of this kind and their use as medicaments, especially as agents for treatment and/or prevention of non-alcoholic fatty liver disease (NAFLD), non-alcoholic steatohepatitis (NASH) and type II diabetes (T2DM).

### Background of the invention

Ketohexokinase (KHK) catalyzes the phosphorylation of fructose to fructose-1-phosphate (F-1-P). Enzymatic activity of human KHK-C in the liver in combination with elevated fructose consumption leads to increased synthesis of fatty acids and trigylcerides. Fructose metabolism by KHK is thought to contribute to a number of diseases like e.g. NAFLD, NASH and T2DM.

Huard et al. (J. Med. Chem. 2017, 60, 7835-7849) describe inhibitors of KHK with a cyanopyridine core, like 6-[(3*S*,4*S*)-3,4-Dihydroxy-1-pyrrolidinyl]-2-[(3*R*)-3-hydroxy-3-methyl-1-pyrrolidinyl]-4-(trifluoromethyl)-3-pyridinecarbonitrile (CAS Registry No. 2711012-28-9).

The aim of the present invention is to provide further cyanopyridine compounds that are more potent KHK inhibitors.

According to the invention, "KHK inhibitor" means a compound which inhibits the enzymatic activity of human ketohexokinase-C as described hereinafter.

### Detailed description of the invention

It has been found that compounds of formula **(I),** wherein the groups **R1** to **R4** have the meanings given hereinafter, surprisingly act as inhibitors of KHK and are capable of inhibiting the generation of fructose-1-phosphate in cells. Thus, the compounds according to the invention may be used for example for the treatment of NAFLD, NASH or T2DM.

The present invention therefore relates to a compound of formula **(I),** or a salt thereof, 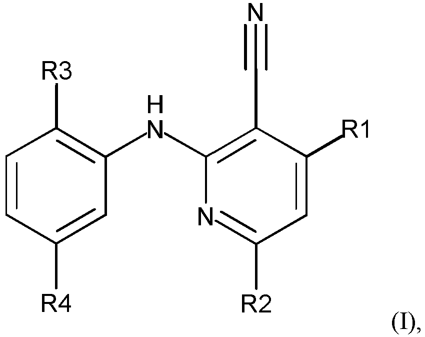 wherein
**R1** is H 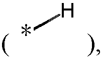 Me 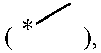 CH₂F 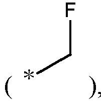 CHF₂ 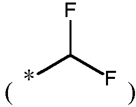 or CF₃ 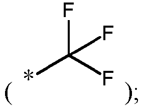
**R2** is selected from the geoup consisting of methylamino 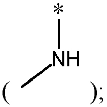 1-pyrrolidinyl 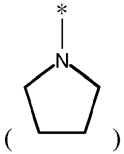 substituted with 1 or two substituents selected from
NH₂ such as in 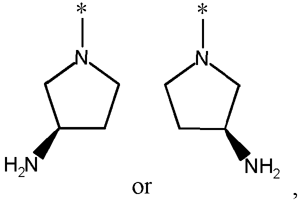
NHMe such as in 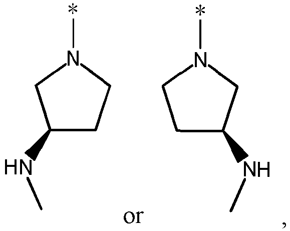
NMe₂ such as in 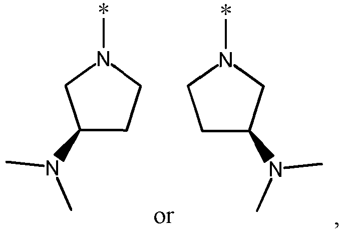
hydroxymethyl such as in 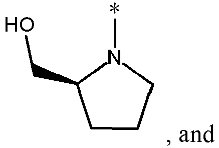
hydroxy (OH) such as in 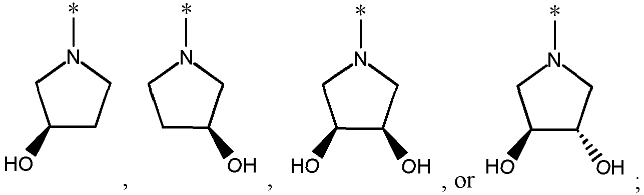 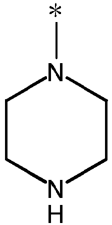 (1-piperazinyl); 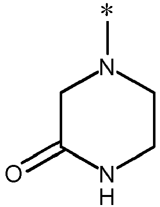 (3-keto-1-piperazinyl) and 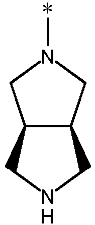 (cis-octahydropyrrolo[3,4-c]pyrrole-2-yl;
**R3** is selected from the group consisting of Cl 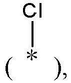 Br 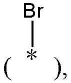 Et 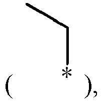 cyclopropyl 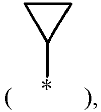 SO₂Me 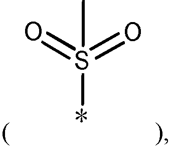 and S**R5** 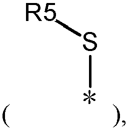 wherein **R5** is Me or Et optionally substituted with up to three F such as in 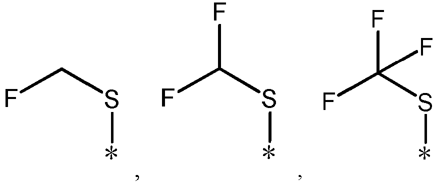 and 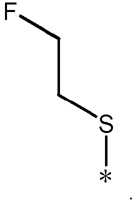
**R4** is selected from the group consisting of H 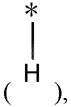 Cl 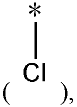 CH₂OH 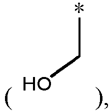 CH₂⁻S(O)-CH₃ ( 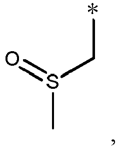 CH₂-CH₂-CONH₂ 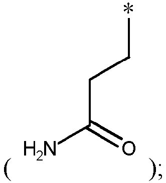 CH₂-COOH 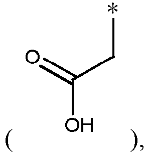 CH₂-CH₂-COOH 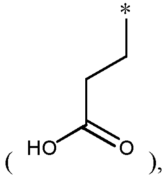 CH₂-CH(Me)-COOH 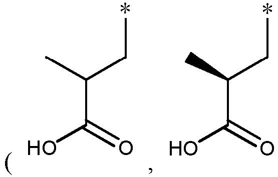 or 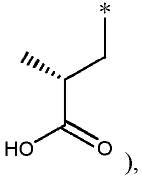 O-CH₂-COOH 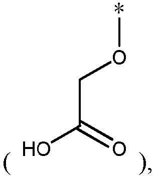 O-CH(Me)-COOH 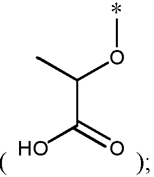 CH₂-P(O)(OH)-OMe 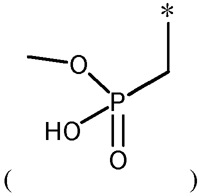 and CH₂-O-P(O)(OH)-Me

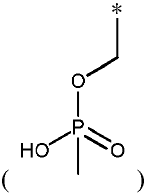

In particular **R1** is preferably CHF₂ 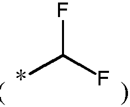 or CF₃

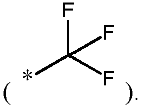

In particular R2 is preferably 3-dimethylamino-pyrrolidin-1-yl such as 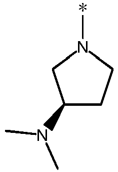 and

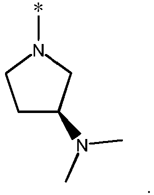

In particular **R3** is preferably SMe 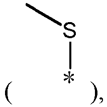 SCHF₂ 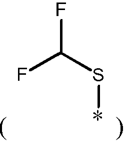 or cyclopropyl

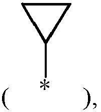

In particular **R4** is preferably CH₂-CH₂-COOH 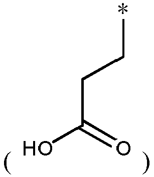 or CH₂-CH(Me)-COOH

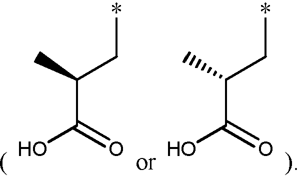

The present invention is directed to compounds of formula **(I)** or salts thereof which are useful in the prevention and/or treatment of a disease and/or condition wherein the inhibition of the ketohexokinase is of therapeutic benefit, including but not limited to the treatment of NAFLD, NASH and T2DM. Thus, in another aspect the invention a compound of formula **(I)** or a pharmaceutically acceptable salt thereof is used as a medicament, and the invention also relates to a compound of formula **(I)** or a pharmaceutically acceptable salt thereof for use in a method of treatment of the human or animal body.

Of particular interest are one of the compounds of formula **(I)** or a salt thereof for use in the treatment of NAFLD, NASH and T2DM, in particular for use in the treatment of NASH. Therefore, another aspect of the invention is to the use of a compound of formula **(I)** or a pharmaceutically acceptable salt thereof for preparing a pharmaceutical composition comprising at least one compound of formula **(I)** or a pharmaceutically acceptable salt thereof and a pharmaceutically acceptable carrier for the treatment of NAFLD, NASH and T2DM. Particularly preferred is their use in preparing a pharmaceutical composition for the treatment of NASH in the human or animal body.

In the treatment of disease like e.g. NAFLD, NASH and T2DM the compound can be administered before, after or together with at least one other pharmacological active substance.

The present invention further relates to a pharmaceutically acceptable salt of a compound of formula **(I)** with an anorganic or organic acid or base. It is expected that a pharmaceutically acceptable salt of a compound of formula **(I)** and/or a co-crystal of a compound of formula **(I),** preferably a pharmaceutically acceptable co-crystal, can be formed.

It can be expected that some compounds of formula **(I)** disclosed herein can form hydrates, solvates, polymorphs, metabolites, derivatives, isomers or prodrugs. Some have chiral centers.

### FORMULATIONS

Suitable preparations for administering the compounds of the invention will be apparent to those with ordinary skill in the art and include for example tablets, pills, capsules, suppositories, lozenges, troches, solutions, elixirs, syrups, sachets, emulsions, inhalatives or dispersible powders. Preferred solutions are solutions for injection (s.c., i.v., i.m.) or solutions for infusion (injectables).

The content of the pharmaceutically active compound needs to be in amounts which are sufficient to achieve the dosage range specified below, for example in the range from 0.1 to 90 wt.-%, preferably 0.5 to 50 wt.-% of the composition as a whole. The doses specified may, if necessary, be given several times a day.

Suitable tablets may be obtained, for example, by mixing the active substance(s) of the invention with known excipients, for example inert diluents, carriers, disintegrants, adjuvants, surfactants, binders and/or lubricants.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may consist of a number of layers. Similarly, the tablet coating may consist of a number of layers to achieve delayed release, possibly using the excipients mentioned above for the tablets.

Syrups or elixirs containing the active substances or combinations thereof according to the invention may additionally contain a sweetener such as saccharine, cyclamate, glycerol or sugar and a flavour enhancer, *e.g.* a flavouring such as vanillin or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection and infusion are prepared in the usual way, *e.g.* with the addition of isotonic agents, preservatives such as p-hydroxybenzoates, or stabilisers such as alkali metal salts of ethylenediamine tetraacetic acid, optionally using emulsifiers and/or dispersants, whilst if water is used as the diluent, for example, organic solvents may optionally be used as solvating agents or dissolving acids, and transferred into injection vials or ampoules or infusion bottles.

Capsules may for example be prepared by mixing the active substance with an inert carriers such as lactose or sorbitol and packing them into gelatine capsules.

Suitable suppositories may be made for example by mixing with carriers provided for this purpose such as neutral fats or polyethyleneglycol or the derivatives thereof.

Excipients which may be used include, for example, water, pharmaceutically acceptable organic solvents such as paraffins (*e.g.* petroleum fractions), vegetable oils (*e.g.* groundnut or sesame oil), mono- or polyfunctional alcohols (*e.g*. ethanol or glycerol), carriers such as *e.g.* natural mineral powders (*e.g.* kaolins, clays, talc, chalk), synthetic mineral powders (*e.g.* highly dispersed silicic acid and silicates), sugars (*e.g*. cane sugar, lactose and glucose), emulsifiers (*e.g.* lignin, spent sulphite liquors, methylcellulose, starch and polyvinylpyrrolidone) and lubricants (*e.g*. magnesium stearate, talc, stearic acid and sodium lauryl sulphate).

The preparations are administered by the usual methods, preferably by an oral or transdermal route, most preferably by oral route. For oral administration the tablets may of course contain, apart from the above-mentioned carriers, additives such as sodium citrate, calcium carbonate and dicalcium phosphate together with various additives such as starch, preferably potato starch, gelatine and the like. Moreover, lubricants such as magnesium stearate, sodium lauryl sulphate and talc may be used at the same time for the tabletting process. In the case of aqueous suspensions the active substances may be combined with various flavour enhancers or colourings in addition to the excipients mentioned above.

For parenteral use, a solution of an active substance with suitable liquid carriers may be used.

The dosage range of a compound of formula **(I)** applicable per day is usually from 1 mg to 2000 mg, preferably from 1 to 1000 mg.

The dosage for intravenous use is from 1 mg to 1000 mg with different infusion rates, preferably between 5 mg and 500 mg with different infusion rates.

It may sometimes be necessary to depart from the amounts specified, depending on the body weight, age, the route of administration, severity of the disease, the individual response to a drug, the nature of the formulation and the time or interval over which the drug is administered (continuous or intermittent treatment with one or multiple doses per day). Thus, in some cases it may be sufficient to use less than the minimum dose given above, whereas in other cases the upper limit may have to be exceeded. When administering large amounts it may be advisable to divide them up into a number of smaller doses spread over the day.

"Pharmaceutically acceptable salts" as used herein refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like.

For example, such salts include salts from benzenesulfonic acid, benzoic acid, citric acid, ethanesulfonic acid, fumaric acid, gentisic acid, hydrobromic acid, hydrochloric acid, maleic acid, malic acid, malonic acid, mandelic acid, methanesulfonic acid, 4-methylbenzenesulfonic acid, phosphoric acid, salicylic acid, succinic acid, sulfuric acid and tartaric acid.

Further pharmaceutically acceptable salts can be formed with cations from ammonia, L-arginine, calcium, 2,2'-iminobisethanol, L-lysine, magnesium, *N*-methyl-D-glucamine, potassium, sodium and tris(hydroxymethyl)-aminomethane.

The pharmaceutically acceptable salts of the present invention can be synthesized from the parent compound which contains a basic or acidic moiety by conventional chemical methods. Generally, such salts can be prepared by reacting the free acid or base form of these compounds with a sufficient amount of the appropriate base or acid in water or in an organic diluent like ether, ethyl acetate, ethanol, isopropanol, or acetonitrile, or a mixture thereof.

### ABBREVIATIONS

| | |
|---|---|
| ACN | Acetonitrile |
| DCM | Dichloromethane |
| DMAP | 4-Dimethylamino-pyridine |
| DMF | N,N,-Dimethylformamide |
| HPLC | High performance liquid chromatography |
| psi | Pounds per square inch |
| RP | Reversed phase |
| SFC | Supercritical fluid chromatography |
| TFA | Trifluoroacetic acid |
| THF | Tetrahydrofuran |
| TLC | Thin layer chromatography |
| XPhos Pd G2 | Chloro(2-dicyclohexylphosphino-2',4',6'-triisopropyl-1,1'-biphenyl)[2-(2'-amino-1,1'-biphenyl)]palladium(II); CAS number 1310584-14-5 |

Features and advantages of the present invention will become apparent from the following detailed examples which illustrate the fundamentals of the invention by way of example without restricting its scope.

### Preparation of the compounds according to the invention

### General

Unless stated otherwise, all the reactions are carried out in commercially obtainable apparatus using methods that are commonly used in chemical laboratories. Starting materials that are sensitive to air and/or moisture are stored under protective gas and corresponding reactions and manipulations therewith are carried out under protective gas (nitrogen or argon).

The compounds according to the invention are named in accordance with IUPAC rules using the software MarvinSketch (Chemaxon). In case of discrepancy between the structure and the name given for a compound, the structure shall prevail.

### Chromatography

Unless otherwise indicated intermediates and final compounds were purified by preparative normal phase chromatography on silica gel using appropriate organic solvents.

NMR Spectra were recorded on a Bruker BBFO ULTRASHIELD^{™}300 AVANCE III 300 MHz or Bruker BBFO ASCEND^{™}400 AVANCE III 400 MHz in the solvent indicated

Anlytical LCMS Methods:

### HPLC Method A:

Column: Sunfire C18_3.0 x 30 mm_2.5 µm
Column producer: Waters

| Time [min] | Vol% water (incl. 0.1% TFA) | Vol% ACN | Flow [ml/min] | Temp [°C] |
|---|---|---|---|---|
| 0.0 | 97 | 3 | 2.2 | 60.0 |
| 0.2 | 97 | 3 | 2.2 | 60.0 |
| 1.2 | 0 | 100 | 2.2 | 60.0 |
| 1.25 | 0 | 100 | 3.0 | 60.0 |
| 1.4 | 0 | 100 | 3.0 | 60.0 |

### HPLC Method B:

Column: Sunfire C18_3.0 x 30 mm_2.5 µm
Column producer: Waters

| Time [min] | Vol% water (incl. 0.1% TFA) | Vol% ACN ( incl. 0.08% TFA (v/v)) | Flow [ml/min] | Temp [°C] |
|---|---|---|---|---|
| 0.0 | 95 | 5 | 1.5 | 60.0 |
| 1.3 | 0 | 100 | 1.5 | 60.0 |
| 1.5 | 0 | 100 | 1.5 | 60.0 |
| 1.6 | 95 | 5 | 1.5 | 60.0 |

### HPLC Method C:

Column: Sunfire C18_2.1 x 30 mm_2.5 µm
Column producer: Waters

| Time [min] | Vol% water (incl. 0.1% TFA) | Vol% ACN | Flow [ml/min] | Temp [°C] |
|---|---|---|---|---|
| 0.0 | 99 | 1 | 1.5 | 60.0 |
| 0.02 | 99 | 1 | 1.5 | 60.0 |
| 1.0 | 0 | 100 | 1.5 | 60.0 |
| 1.1 | 0 | 100 | 1.5 | 60.0 |

### HPLC Method D:

Column: XBridge C18_3.0 x 30 mm_2.5 µm
Column producer: Waters

| Time [min] | Vol% water (incl. 0.1% NH₃) | Vol% ACN | Flow [ml/min] | Temp [°C] |
|---|---|---|---|---|
| 0.0 | 97 | 3 | 2.2 | 60.0 |
| 0.2 | 97 | 3 | 2.2 | 60.0 |
| 1.2 | 0 | 100 | 2.2 | 60.0 |
| 1.25 | 0 | 100 | 3.0 | 60.0 |
| 1.4 | 0 | 100 | 3.0 | 60.0 |

### HPLC Method E:

Column: Sunfire C18_3.0 x 30 mm_2.5 µm
Column producer: Waters

| Time [min] | Vol% water (incl. 0.1% TFA) | Vol% ACN (incl. 0.08% TFA (v/v)) | Flow [ml/min] | Temp [°C] |
|---|---|---|---|---|
| 0.0 | 95 | 5 | 1.5 | 60.0 |
| 1.3 | 0 | 100 | 1.5 | 60.0 |
| 1.5 | 0 | 100 | 1.5 | 60.0 |
| 1.6 | 95 | 5 | 1.5 | 60.0 |

### HPLC Method F:

Column: XBridge C18_3.0 x 30 mm_2.5 µm
Column producer: Waters

| Time [min] | Vol% water (incl. 0.1% NH₃) | Vol% ACN | Flow [ml/min] | Temp [°C] |
|---|---|---|---|---|
| 0.0 | 95 | 5 | 1.5 | 60.0 |
| 1.3 | 0 | 100 | 1.5 | 60.0 |
| 1.5 | 0 | 100 | 1.5 | 60.0 |
| 1.6 | 95 | 5 | 1.5 | 60.0 |

### HPLC Method G:

Column: Sunfire C18_3.0 x 30 mm_2.5 µm
Column producer: Waters

| Time [min] | Vol% water (incl. 0.1% TFA) | Vol% ACN (incl. 0.08% TFA (v/v)) | Flow [ml/min] | Temp [°C] |
|---|---|---|---|---|
| 0.0 | 95 | 5 | 1.5 | 60.0 |
| 1.3 | 0 | 100 | 1.5 | 60.0 |
| 1.5 | 0 | 100 | 1.5 | 60.0 |
| 1.6 | 95 | 5 | 1.5 | 60.0 |

### HPLC Method H:

Column: XBridge C18_3.0 x 30 mm_2.5 µm
Column producer: Waters

| Time [min] | Vol% water (incl. 0.1% NH₃) | Vol% ACN | Flow [ml/min] | Temp [°C] |
|---|---|---|---|---|
| 0.0 | 95 | 5 | 1.5 | 60.0 |
| 1.3 | 0 | 100 | 1.5 | 60.0 |
| 1.5 | 0 | 100 | 1.5 | 60.0 |
| 1.6 | 95 | 5 | 1.5 | 60.0 |

### HPLC Method I:

Column: Sunfire C18_3.0 x 30 mm_2.5 µm
Column producer: Waters

| Time [min] | Vol% water (incl. 0.1% TFA) | Vol% ACN (incl. 0.08% TFA (v/v)) | Flow [ml/min] | Temp [°C] |
|---|---|---|---|---|
| 0.0 | 95 | 5 | 1.5 | 60.0 |
| 1.3 | 0 | 100 | 1.5 | 60.0 |
| 1.5 | 0 | 100 | 1.5 | 60.0 |
| 1.6 | 95 | 5 | 1.5 | 60.0 |

The compounds according to the invention are prepared by the methods of synthesis described hereinafter in which the substituents of the general formulae have the meanings given hereinbefore. These methods are intended as an illustration of the invention without restricting its subject matter and the scope of the compounds claimed to these examples. Where the preparation of starting compounds is not described, they are commercially obtainable or may be prepared analogously to known compounds or methods described herein. Substances described in the literature are prepared according to the published methods of synthesis.

The syntheses described hereinafter may produce the respective compounds in the salt form (e.g. free base or acid) as indicated or in a different salt form (e.g. TFA salt).

### A. General preparation methods for compounds of formula (I)

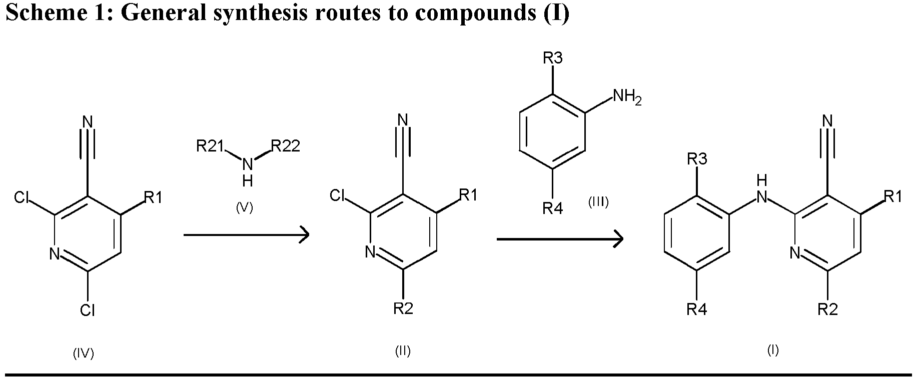

Compounds of general formula (I) can be prepared by reacting chloropyridine compounds of general formula (II) with anilins of general formula (III) under standard cross-coupling conditions applying a palladium catalyst like e.g. XPhos Pd G2 and a base like e.g. cesium carbonate in a solvent like e.g. dioxane. The substituents R3 and R4 in anilins (III) may thereby be beyond the claimed scope of the invention and be converted into substituents within the scope of this invention later (e.g. by removal of protecting groups). Compounds of general formula (II) can be prepared from dichloropyridines of general formula (IV) through reaction with an approprioate primary or secondary amine (V) in presence of a base like e.g. N,N-diisopropylethylamine in a solvent like e.g. ethanol. The structure of the amine (V) applied may thereby be beyond the claimed scope of the invention and be converted into a structure R2 that is within the scope of this invention later (e.g. by removal of protecting groups). Dichloropyridine compounds of general formula (IV) can be prepared by methods known in the literature or described hereinafter.

### B. Synthesis of intermediates

### B.1 Synthesis of dichloropyridine intermediates

### Intermediate I.1: 2,6-dichloro-4-(fluoromethyl)pyridine-3-carbonitrile

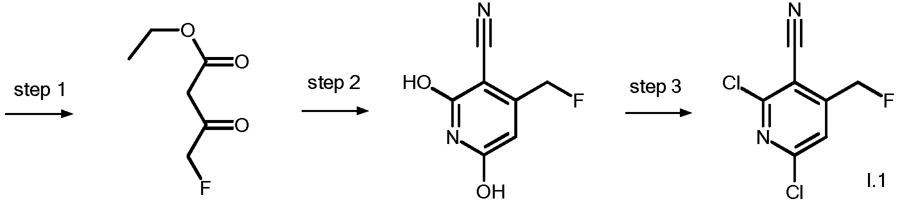

### Step 1:

Lithium diisopropylamide (100 ml; 2 mol/l in THF; 200 mmol) was cooled to -70°C. Acetic acid ethyl ester (19.6 ml; 200 mmol) was added and the mixture was stirred at -70 °C for 1 hour. Ethyl fluoroacetate (17.5 ml; 180 mmol) was added dropwise, the mixture was stirred for another 45 min then allowed to warm to ambient temperature. Ethyl acetate (100 ml) was added, and the mixture was acidified by addition of aq. HCl (4 mol/l). The organic layer was separated, washed with brine, dried with magnesium sulphate, and evaporated to dryness to yield the crude product ethyl 4-fluoro-3-oxobutanoate as a brownish oil (24.7g; 93%) which was taken to the next step without further purification.

### Step 2:

To a mixture of the crude product from step 1 (24.7 g) and cyanoacetamide (14.0 g; 167 mmol) in ethanol (100 ml) was added KOH (9.36 g; 167 mmol). The mixture was refluxed over night, then the precipitate formed was filtered off with suction. The solid was taken up in warm water (30 ml) and acidified by addition of aq. HCl (4 mol/l). The precipitate was filtered off with suction, washed with water and dried at 50 °C to yield 2,6-dihydroxy-4-(fluoromethyl)pyridine-3-carbonitrile (18.7g; 111 mmol; 67%) as a slightly brownish solid. MS (ESI pos.+neg. Loop-Inj.) *m*/*z:* 169 [M + H]⁺

### Step 3:

A mixture of 2,6-dihydroxy-4-(fluoromethyl)pyridine-3-carbonitrile from step 2 (6.0 g; 35.7 mmol) and trichlorophosphate (50 ml; 546 mmol) in a closed vessel was heated to 140 °C over night, then allowed to cool to ambient temperature. The mixture was added dropwise to warm water (400 ml) not exceeding a temperature of 50 °C. Caution! Exothermic! The resulting mixture was cooled to 0 °C, the precipitate formed was filtered off with suction, washed with water and dried at ambient temperature to yield 2,6-dichloro-4-(fluoromethyl)pyridine-3-carbonitrile (4.81 g; 23.5 mmol; 66%) as a brownish powder. ¹H NMR (400 MHz, DMSO-*d*₆) δ ppm 7.87 (s, 1 H), 5.72 (d, *J*=45.73 Hz, 2 H) MS (ESI pos.+neg. Loop-Inj.) *m*/*z:* 203 [M-H]⁻

### Intermediate 1.2: 2,6-dichloro-4-(difluoromethyl)pyridine-3-carbonitrile

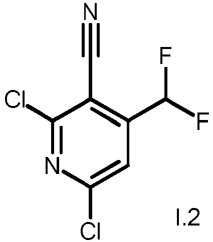

Was prepared analogously to the procedure described for the synthesis of intermediate I.1, starting from 4-difluoro-3-oxobutanoate, applying step 2 and step 3.

¹H NMR (400 MHz, DMSO-*d*₆) δ ppm 8.09 (s, 1 H), 7.29 (t, *J*=53.05 Hz, 1 H) MS (ESI pos.+neg. Loop-Inj.) *m*/*z:* 221 [M - H]⁻; *m*/*z:* 203 [M+H2O-HCl-H]⁻

### B.2 Synthesis of anilin intermediates

### Intermediate II.1: methyl 2-[3-amino-4-(methylsulfanyl)phenyl]acetate

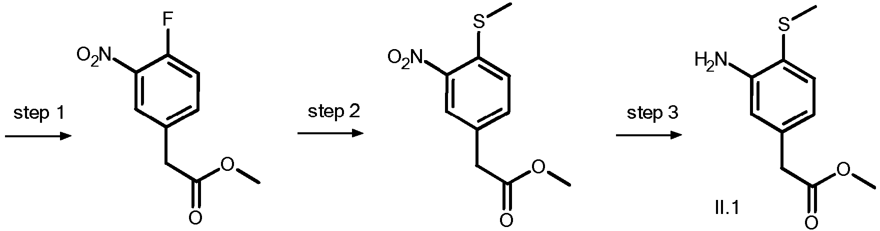

### Step 1:

To a stirred mixture of 2-(4-fluoro-3-nitrophenyl)acetic acid (6.00 g; 30 mmol) inDMF (30 ml) was added NaOH (1.33 g; 33 mmol) followed by water (8.0 ml) at room temperature. Methyl mercaptan sodium salt (15% in water; 2.32 g; 33 mmol) was added drop wise to the reaction mixture, while temperature raised to 50 °C. After 30mins, 20ml of cold water followed by 20ml of 4M HCl solution was added to the reaction mixture, the resulting yellow solid was collected by filtration, washed with water, dried under vaccum at 50 °C to provide 2-[4-(methylsulfanyl)-3-nitrophenyl]acetic acid (6.00g; 22 mmol; 88%) as a yellow solid.

### Step 2:

To a solution of the product obtained from step 1 in methanol (100 ml) was added concentrated sulfuric acid (1.2 ml) at room temperature. The mixture was stirred for 6 hour at reflux temperature, then volatiles were evaporated. The resulting mixture was diluted with ethyl acetate, washed first with saturated sodium bicarbonate solution, then with brine, dried over magnesium sulfate. The filtrated organic layer was evaporated to dryness to yield crude methyl 2-[4-(methylsulfanyl)-3-nitrophenyl]acetate (7.0g) which was taken to the following step without purification.

### Step 3:

To a degassed solution of the crude product from step 2 (6.0 g; 25 mmol) in methanol (40 ml) was added palladium 10% on carbon (2.65 g). The mixture was stirred in a parr shaker under 50 psi hydrogen pressure for 12 hours. The reaction mixture was filtered through celite and concentrated under reduced pressure. The crude product was purified by silica gel chromatography (DCM/Methanol) to yield methyl 2-[3-amino-4-(methylsulfanyl)phenyl]acetate (1.50 g; 7.10 mmol; 29%) as a colourless liquid.
MS (ESI pos.+neg. Loop-Inj.) *m*/*z:* 212 [M + H]⁺

### Intermediate II.2: ethyl 3-[3-amino-4-(methylsulfanyl)phenyl]-2-methylpropanoate

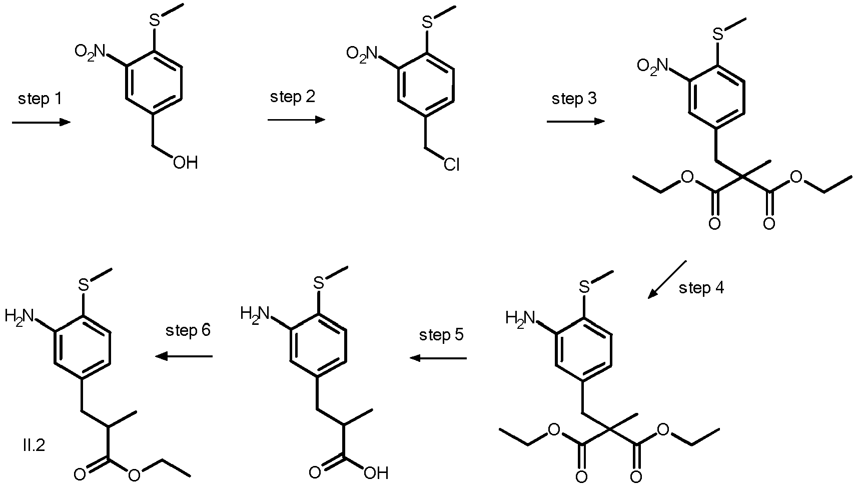

### Step 1:

To a solution of 4-chloro-3-nitrobenzyl alcohol (11.0 g; 58.6 mmol) in DMF (250 ml) was added slowly sodium methanethiolate (15% in water; 49.8 ml; 117 mmol). The mixture was stirred over night, then water was added and the mixture was acidified by addition of aq. HCl. The precipitate formed was filtered off with suction and dried at 50 °C to yield [3-amino-4-(methylsulfanyl)phenyl]methanol (9.50 gg; 81%) as a yellow solid.
MS (ESI pos.+neg. Loop-Inj.) *m*/*z:* 200 [M + H]⁺
RT = 0.51 min (HPLC method C)

### Step 2:

The product of step 1 (9.50 g; 37.7 mmol) was dissolved in thionyl chloride (100 ml). The mixture was stirred for 3 hours at ambient temperature, then evaporated to dryness and coevaporated with diethyl ether to yield 5-(chloromethyl)-2-(methylsulfanyl)aniline (10.0 g; 96%) as a yellow solid.
RT = 0.77 min (HPLC method C)

### Step 3:

Under Argon! To a solution of sodium hydride (60% in mineral oil; 4.04 g; 101 mmol in DMF (50 ml) was added dropwise while cooling with an ice-bath a solution of diethyl methylmalonate (15.7 ml; 92 mmol) in DMF (30 ml). After further 10 min stirring, a solution of the product from step 2 (10.0 g; 46 mmol) in DMF (20 ml) was added dropwise. The mixture was stirred for 1 h at ambient temperature, then poured on ice-water and extracted with DCM. The organic layer was separated, washed with brin and dried over magnesium sulphate, filtrated and evaporated to yield 1,3-diethyl 2-methyl-2-{[4-(methylsulfanyl)-3-nitrophenyl]methyl}propanedioate (16.6 g; 86%) as a dark brown oil.
¹H NMR (400 MHz, DMSO-*d*₆) δ ppm 8.00 (d, *J*=1.27 Hz, 1 H), 7.49 - 7.56 (m, 2 H), 4.10 - 4.19 (m, 4 H), 3.22 (s, 2 H), 2.52 (s, 3 H), 1.27 (s, 3 H), 1.19 (t, *J*=7.10 Hz, 6 H)
MS (ESI pos.+neg. Loop-Inj.) *m*/*z:* 356 [M + H]⁺
RT = 1.13 min (HPLC method A)

### Step 4:

To the product from step 3 (3.24 g; 9.11 mmol) in THF (40 ml) was added Raney-Nickel (400 mg). The mixture was shaken under 50 psi hydrogen until TLC indicated complete consumption of starting material. The catalyst was filtered off and the filtrate was evaporated to yield 1,3-diethyl 2-{[3-amino-4-(methylsulfanyl)phenyl]methyl }-2-methylpropanedioate (2.79 g; 94%).
¹H NMR (400 MHz, DMSO-*d*₆) δ ppm 7.07 (d, *J*=7.83 Hz, 1 H), 6.43 (d, *J*=1.52 Hz, 1 H), 6.29 (dd, *J*=7.83, 1.77 Hz, 1 H), 5.11 (s, 2 H), 4.13 (q, *J*=7.07 Hz, 4 H), 2.95 (s, 2 H), 2.28 (s, 3 H), 1.21 (s, 3 H), 1.18 (t, *J*=7.07 Hz, 6 H)
MS (ESI pos.+neg. Loop-Inj.) *m*/*z:* 326 [M + H]⁺
RT = 1.02 min (HPLC method A)

### Step 5:

A mixture oft he product from step 4 (2.72 g; 8.58 mmol), dioxane (30 ml) and aq. HCl (4 mol/l; 15 ml) was refluxed for 24 hours. The mixure was evaporated and coevaporated with toluene added to yield crude 3-[3-amino-4-(methylsulfanyl)phenyl]-2-methylpropanoic acid (2.34 g; 121%) which was taken to the next step without purification.

### Step 6:

To a solution of crude product from step 5 3.14 g; 13.9 mmol) in ethanol (40 ml) was added drowise thionyl chloride (1.98 g; 1.21 ml; 16.7 mmol). Caution: exothermic reaction! The mixture was stirred over night, evaporated, taken up in ethyl acetate and extracted with potassium carbonate (10% aq. Solution). The aqueous layer was reextracted with ethyl acetate. The combined organic layers were extracted with water and then with brine, separated, dried over magnesiium sulphate, filtered and evaporated. The crude product was purified by silica gel chromatography (petrol ether / ethyl acetate 5%-> 25%) to yield ethyl 3-[3-amino-4-(methylsulfanyl)phenyl]-2-methylpropanoate (1.08 g; 31%) as a brownish oil. ¹H NMR (400 MHz, DMSO-*d*₆) δ ppm 7.10 (d, *J*=7.86 Hz, 1 H), 6.51 (d, *J*=1.77 Hz, 1 H), 6.36 (dd, *J*=7.86, 1.90 Hz, 1 H), 5.11 (s, 2 H), 4.02 (q, *J*=7.05 Hz, 2 H), 2.69 - 2.77 (m, 1 H), 2.57 - 2.68 (m, 1 H), 2.45 - 2.51 (m, 1 H), 2.26 (s, 3 H), 1.12 (t, *J*=7.10 Hz, 3 H), 1.05 (d, *J*=6.84 Hz, 3 H)
MS (ESI pos.+neg. Loop-Inj.) *m*/*z:* 254 [M + H]⁺
RT = 0.97 min (HPLC method A)

### Intermediate II.3: 3-[3-amino-4-(methylsulfanyl)phenyl]propanoic acid

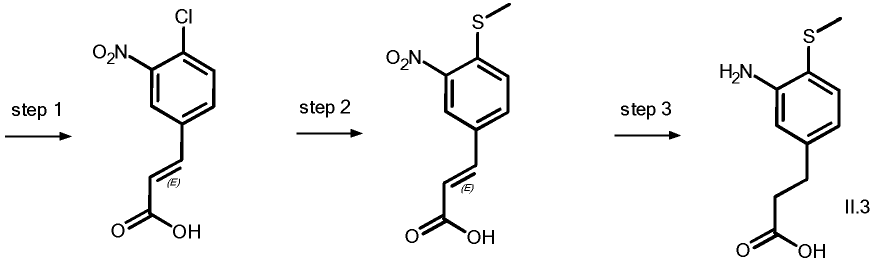

### Step 1:

To a solution of 4-chloro-3-nitrocinnamic acid (1.00g; 8.79 mmol) in DMF (30 ml) was added sodium hydroxide (387 mg; 9.67 mmol). The mixture was stirred until all NaOH was dissolved, then sodium methanethiolate (15% in water; 4.11 ml; 9.67 mmol) was added, and the mixture was stirred for another 2 hours. Ice-water (30 ml) was added, then the mixture was acidified by addition of aq. HCl (6 mol/l; 3.5 ml). The precipitate formed was filtered off with suction, washed with water and dries at 50 °C to yield 3-[4-(methylsulfanyl)-3-nitrophenyl]prop-2-enoic acid (2.05 g; 98%) as a yellow solid.
MS (ESI pos.+neg. Loop-Inj.) *m*/*z:* 238 [M - H]⁻
RT = 0.94 min (HPLC method A)

### Step 2:

To a solution of the product from step 1 (0.300 g; 1.25 mmol) in methanol (10 ml) was added palladium on charcoal (10%). The mixture was shaken under 50 psi hydrogen pressure over night. The catalyst was filtered off with suction and the filtrate was evaporated to dryness. The residue was taken up in methanol (10 ml), and raney nickel (100 mg) was added. The mixture was shaken under 50 psi hydrogen pressure for 3 days. The catalyst was filtered off with suction and the filtrate was evaporated to dryness. The crude product was purified by preparative RP-HPLC (column: C18; eluent: water/ACN/TFA) to yield the title compound (85 mg; 22%) as a slightly brownish solid.
MS (ESI pos.+neg. Loop-Inj.) *m*/*z:* 212 [M - H]⁻
RT = 0.82 min (HPLC method C)

### C. Synthesis of example compounds

### Example 1

### 2-[3-({6-[cis-octahydropyrrolo[3,4-c]pyrrol-2-yl]-3-cyano-4-(trifluoromethyl)pyridin-2-yl}amino)-4-(methylsulfanyl)phenyl]acetic acid

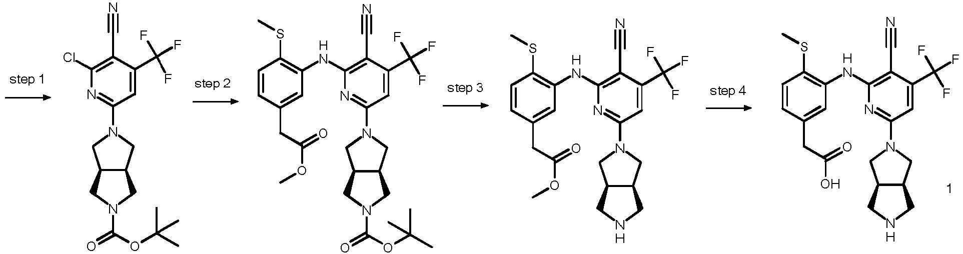

### Step 1 (Procedure A)

To a solution of the dichloropyridine reagent 2,6-dichloro-4-(trifluoromethyl)nicotinonitrile (1.70g; 7.05 mmol) in ethanol (50 ml) were added the amine reagent cis-N-BOC-hexahydro-pyrrolo[3,4-c]pyrrole (1.50g; 7.05 mmol) and N,N-diisopropylethylamine (2.44 ml; 14.1 mmol). The mixture was stirred over night at ambient temperature and evaporated to dryness. The crude product was purified by preparative RP-HPLC (column: C18 X-bridge; mobile phase: water-ACN + 0.1% aq. Ammonia; 55 °C) to afford tert-butyl 5-[6-chloro-5-cyano-4-(trifluoromethyl)pyridin-2-yl]-cis-octahydropyrrolo[3,4-c]pyrrole-2-carboxylate (1.98 g; 67%) as a colourless solid.
MS (ESI pos.+neg. Loop-Inj.) *m*/*z:* 417 [M + H]⁺
RT = 1.22 min (HPLC method A)

### Step2 (Procedure B)

To a mixture of tert-butyl 5-[6-chloro-5-cyano-4-(trifluoromethyl)pyridin-2-yl]-cis-octahydropyrrolo[3,4-c]pyrrole-2-carboxylate (from step 1; 50 mg; 0.12 mmol), the catalyst XPhos Pd G2 (20 mg; 0.025 mmol) and cesium carbonate (110 mg; 0.34 mmol) in dioxane (2.0 ml) was added the aniline reagent intermediate II.1 (methyl 2-[3-amino-4-(methylsulfanyl)phenyl]acetate; 30 mg; 0.14 mmol). The mixture was stirred at 100°C for 4h, allowed to cool to ambient temperature, diluted with DMF, filtered and evaporated to dryness. The crude product was purified by preparative RP-HPLC (column: C18; mobile phase: water-ACN + TFA; 60 °C).

### Step 3 (Acidic deprotection; procedure C)

The product from step 2 was taken up in DCM/TFA (3:1) and stirred for 2h at ambient temperature. The mixture was evaporated to dryness to afford methyl 2-[3-({6-[cis-octahydropyrrolo[3,4-c]pyrrol-2-yl]-3-cyano-4-(trifluoromethyl)pyridin-2-yl}amino)-4-(methylsulfanyl)phenyl]acetate as a TFA salt (29 mg; 40% over two steps).
MS (ESI pos.+neg. Loop-Inj.) *m*/*z:* 492 [M + H]⁺
RT = 0.96 min (HPLC method A)

### Step 4 (Basic deprotection; procedure D)

To a solution of methyl 2-[3-({6-[cis-octahydropyrrolo[3,4-c]pyrrol-2-yl]-3-cyano-4-(trifluoromethyl)pyridin-2-yl}amino)-4-(methylsulfanyl)phenyl] acetate (from step 3; 25 mg; 0.041 mmol) in methanol (1.0 ml) was added aq. NaOH (1 mol/l; 150 µl; 0.15 mmol). The mixture was stirred over night at ambient temperature, then acidified by addicition of aq. HCl (1 mol/l; 150 µl). The product was purified by preparative RP-HPLC (column: C18; mobile phase: water-ACN + TFA; 60 °C) to yield 2-[3-({6-[cis-octahydropyrrolo[3,4-c]pyrrol-2-yl]-3-cyano-4-(trifluoromethyl)pyridin-2-yl}amino)-4-(methylsulfanyl)phenyl]acetic acid as a TFA salt (21 mg; 86%).
¹H NMR (400 MHz, DMSO-*d*₆) δ ppm 8.86 (br s, 2 H), 8.59 (s, 1 H), 7.95 (d, *J*=1.65 Hz, 1 H), 7.43 (d, *J*=7.98 Hz, 1 H), 7.06 (dd, *J*=8.05, 1.84 Hz, 1 H), 6.35 (s, 1 H), 3.65 - 3.74 (m, 2 H), 3.57 (s, 2 H), 3.40 - 3.55 (m, 4 H), 3.07 - 3.16 (m, 4 H), 2.40 (s, 3 H)
MS (ESI pos.+neg. Loop-Inj.) *m*/*z:* 478 [M + H]⁺
RT = 0.92 min (HPLC method A)

### Example 2

### 3-(3-{[3-cyano-4-(difluoromethyl)-6-[(3S)-3-(dimethylamino)pyrrolidin-1-yl]pyridin-2-yl]amino}-4-(methylsulfanyl)phenyl)-2-methylpropanoic acid

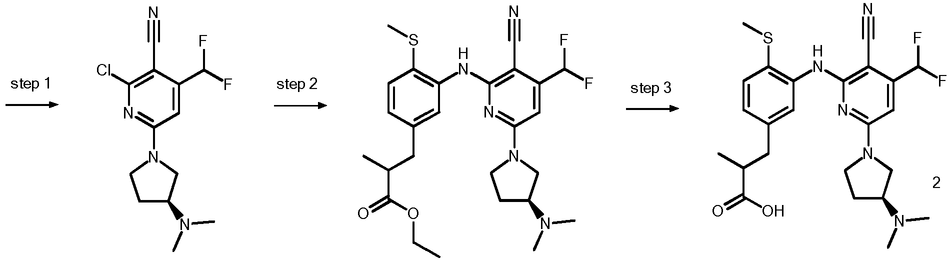

Step 1 was performed according to procedure A, applying the dichloropyridine reagent 2,6-dichloro-4-(difluoromethyl)pyridine-3-carbonitrile and the amine reagent (3S)-N,N-dimethylpyrrolidin-3-amine to yield 2-chloro-4-(difluoromethyl)-6-[(3S)-3-(dimethylamino)pyrrolidin-1-yl]pyridine-3-carbonitrile as a light brown solid.
MS (ESI pos.+neg. Loop-Inj.) *m*/*z:* 301 [M + H]⁺
RT = 0.97 min (HPLC method D)

Step 2 was performed according to procedure B (but without chromatographic purification), applying the aniline reagent ethyl 3-[3-amino-4-(methylsulfanyl)phenyl]-2-methylpropanoate (intermediate II.1).

The crude product from step 2 was taken to
step 3 which was performed according to procedure D to yield 3-(3-{[3-cyano-4-(difluoromethyl)-6-[(3S)-3-(dimethylamino)pyrrolidin-1-yl]pyridin-2-yl]amino}-4-(methylsulfanyl)phenyl)-2-methylpropanoic acid as a TFA salt (as a slightly brownish solid).
MS (ESI pos.+neg. Loop-Inj.) *m*/*z:* 490 [M + H]⁺
RT = 0.88 min (HPLC method A)

### Examples 3 and 4

Chiral separation of example 2 (3-(3-{[3-cyano-4-(difluoromethyl)-6-[(3S)-3-(dimethylamino)pyrrolidin-1-yl]pyridin-2-yl]amino}-4-(methylsulfanyl)phenyl)-2-methylpropanoic acid)

The diastereomeric mixture example 2 was separated applying chiral SFC:
Column: CHIRAL ART^{®} Cellulose-SC 20 x 250 mm 5 µm
Solvents: scCO2 75 %; 2-propanol +20mM NH3 25 %
Backpressure regulator 150 bar
Temperature 40 °C
Flowrate 60 ml/min
Sample concentration 20 mg/ml
Sample solvent methanol
Injection volume 200 µl

The two isolated crude diastereomers were further purified by preparative RP-HPLC (column: C18; mobile phase: water-ACN + TFA; 60 °C) and lyophilized to yield the title compounds as TFA salts (slightly yellowish solids).

Chiral purity was determined by analytic SFC. Column: CHIRAL ART^{®} Cellulose-SC; solvents: scCO2 75 %; 2-propanol +10mM NH3 25 %

### Example 3 (diastereomer eluting first; RT = 3.16 min):

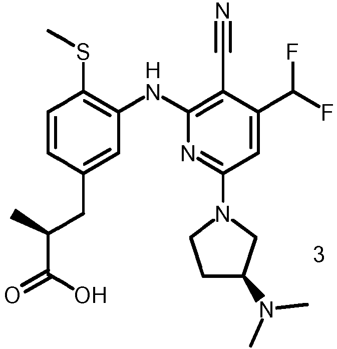

The absolute configuration of the methyl substituent in alpha position to the carboxylic acid was assigned arbitrarily, the pure diastereomer example 3 may therefore have the structure as shown or the structure shown for example 4.
MS (ESI pos.+neg. Loop-Inj.) *m*/*z:* 490 [M + H]⁺
RT = 0.90 min (HPLC method A)

### Example 4 (diastereomer eluting second; RT = 3.61 min) :

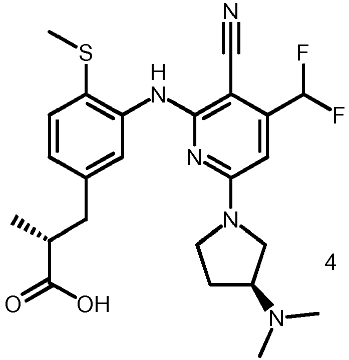

The absolute configuration of the methyl substituent in alpha position to the carboxylic acid was assigned arbitrarily, the pure diastereomer example 4 may therefore have the structure as shown or the structure shown for example 3.
MS (ESI pos.+neg. Loop-Inj.) *m*/*z:* 490 [M + H]⁺
RT = 0.89 min (HPLC method A)

### Preparation of example 4 in salt-free (zwitterionic) form:

Example 4, prepared as described above, (TFA salt; 2.4 g) was taken up in water-ethanol (3:1; 800 ml). The mixture was heated to 90 °C and filtered hot. Through addition of aq. NaOH (1 mol/l) the pH was adjusted to 6-7, then the solution was allowed to cool to ambient temperature and stand over night. The precipitate was filtered off, washed with water and dried at 60 °C to yield 1.27g (69%) of the zwitterionic form as off-white, amorphous solid. ¹H NMR (400 MHz, DMSO-*d*₆) δ ppm 8.43 (s, 1 H), 8.14 (s, 1 H), 7.41 (d, *J*=7.98 Hz, 1 H), 7.00 (t, *J*=54.20 Hz, 1 H), 6.95 (dd, *J*=7.98, 1.52 Hz, 1 H), 6.21 - 6.36 (m, 1 H), 3.53 - 3.88 (m, 3 H), 3.13 - 3.49 (m, 3 H), 2.78 - 2.94 (m, 2 H), 2.54 - 2.69 (m, 2 H), 2.38 (s, 3 H), 2.20 (s, 6 H), 1.05 (d, *J*=6.59 Hz, 3 H).

The following examples of Table 1a were prepared by analogous procedures from starting materials as indicated in Table 1b applying procedures described above (see syntheses of examples 1 and 2) in reaction sequences as indicted:

**Table 1a:**

| **Example #** | **Structure** | **[M+H]⁺ [m/z]** | **HPLC RT [min] (HPLC method)** |
|---|---|---|---|
| **5** | 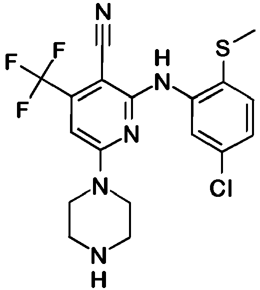 | 428 | 1.00 min (H) |
| **6** | 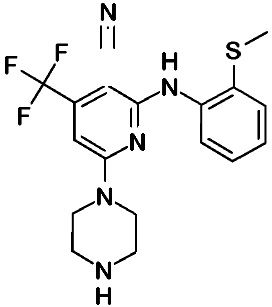 | 394 | 0.90 min (H) |
| **7** | 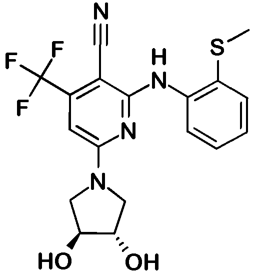 | 411 | 0.87 min (G) |
| **8** | 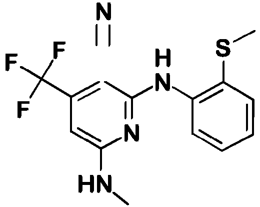 | 339 | 1.05 min (B) |
| **9** | 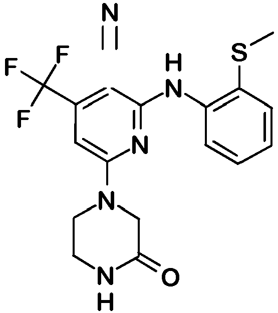 | 408 | 0.71 min (C) |
| **10** | 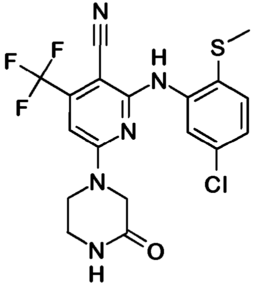 | 442 | 1.10 min (D) |
| **11** | 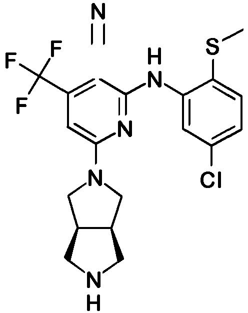 | 454 | 0.75 min (E) |
| **12** | 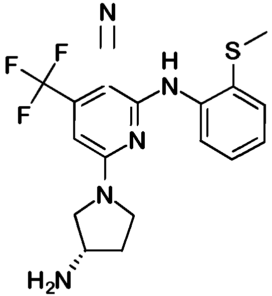 | 394 | 0.69 min (G) |
| **13** | 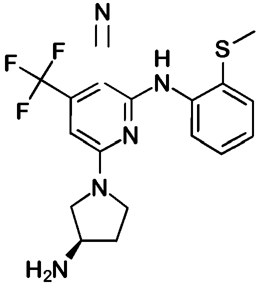 | 394 | 0.69 min (G) |
| **14** | 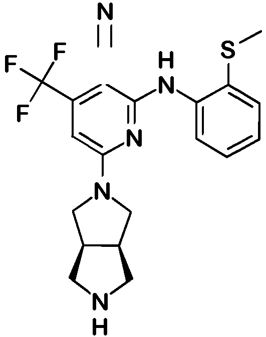 | 420 | 0.70 min (E) |
| **15** | 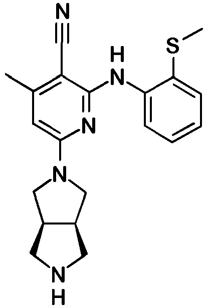 | 366 | 0.93 min (F) |
| **16** | 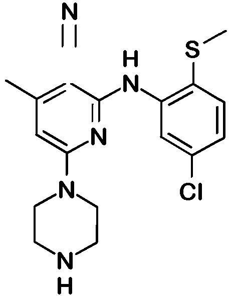 | 374 | 0.91 min (A) |
| **17** | 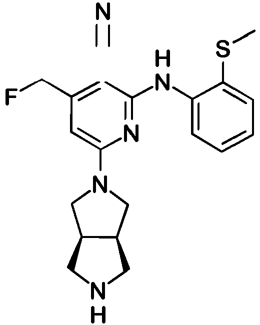 | 384 | 0.63 min (E) |
| **18** | 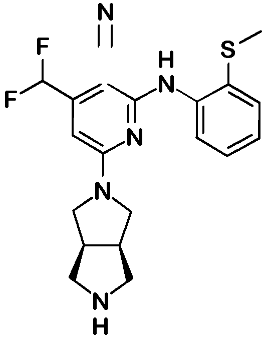 | 402 | 0.64 min (E) |
| **19** | 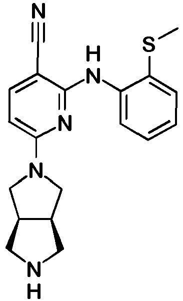 | 352 | 0.87 min (A) |
| **20** | 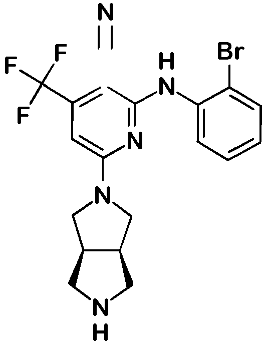 | 352 | 1.12 (D) |
| **21** | 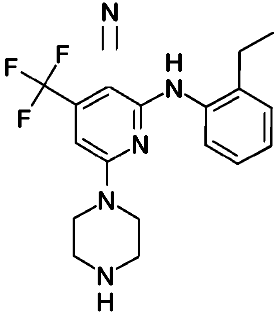 | 376 | 0.55 min (C) |
| **22** | 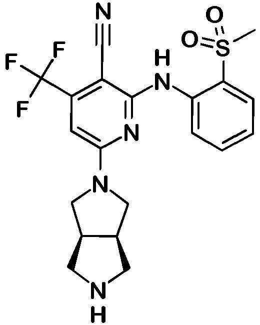 | 452 | 0.59 min (E) |
| **23** | 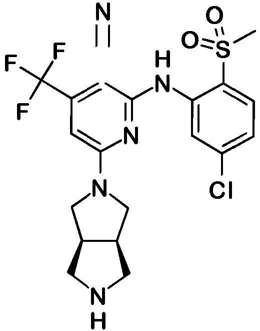 | 486 | 0.53 (C) |
| **24** | 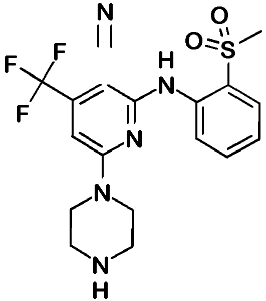 | 426 | 0.97 min (D) |
| **25** | 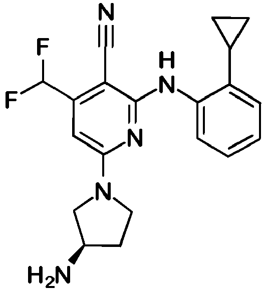 | 370 | 0.68 (E) |
| **26** | 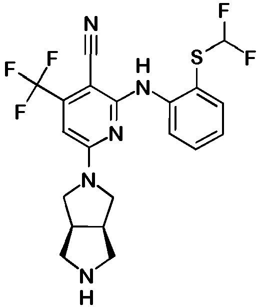 | 456 | 0.70 min (E) |
| **27** | 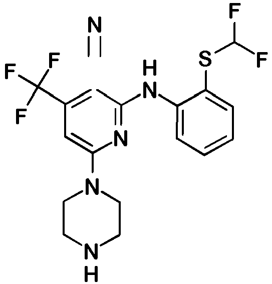 | 430 | 0.56 min (C) |
| **28** | 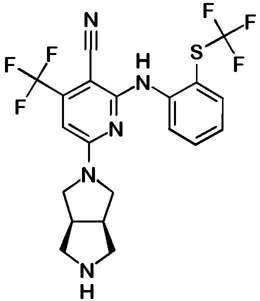 | 474 | 0.70 min (G) |
| **29** | 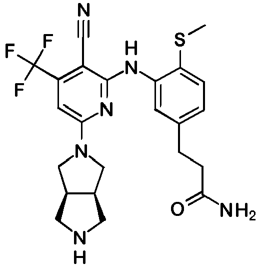 | 491 | 0.81 min (H) |
| **30** | 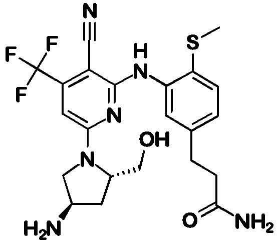 | 495 | 0.68 min (H) |
| **31** | 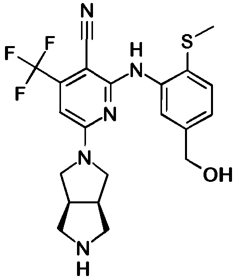 | 450 | 0.54 min (C) |
| **32** | 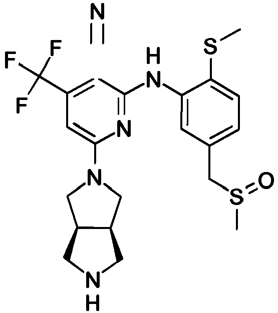 | 496 | 0.63 min (G) |
| **33** | 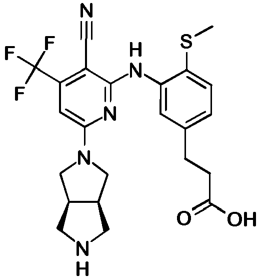 | 492 | 0.70 min (G) |
| **34** | 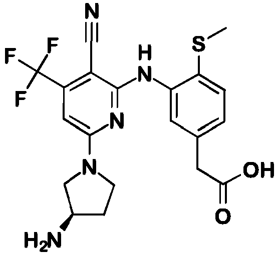 | **452** | 0.91 min (A) |
| **35** | 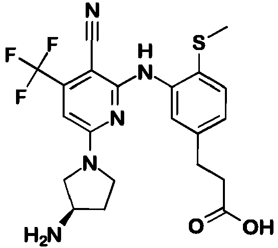 | 466 | 0.67 min (E) |
| **36** | 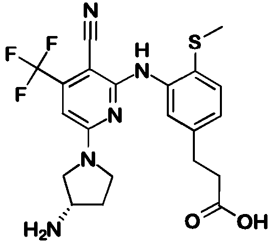 | 466 | 0.67 min (E) |
| **37** | 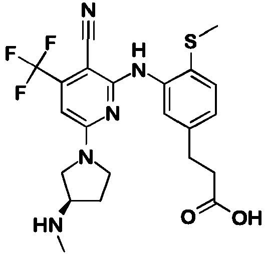 | 480 | 0.67 min (E) |
| **38** | 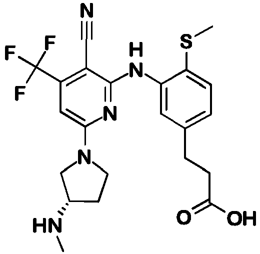 | 480 | 0.66 (E) |
| **39** | 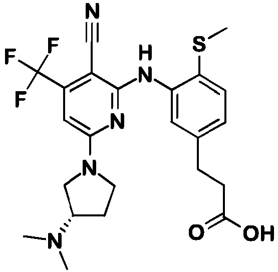 | 494 | 0.93 min (A) |
| **40** | 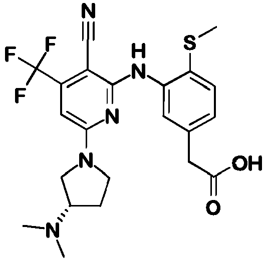 | 480 | 0.51 min (H) |
| **41** | 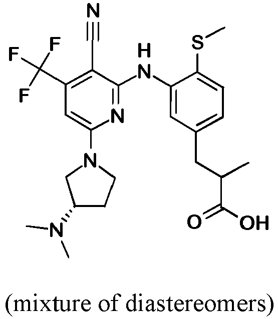 | 508 | 0.91 min (A) |
| **42** | 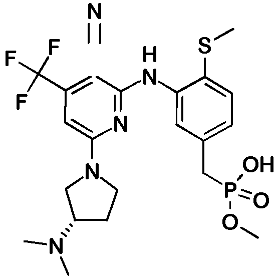 | 530 | 0.91 min (A) |
| **43** | 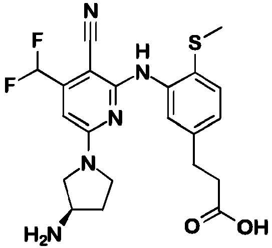 | 448 | 0.64 min (I) |
| **44** | 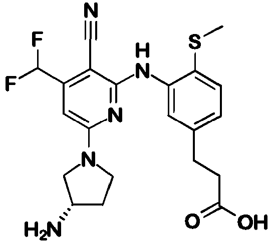 | 448 | 0.64 min (I) |
| **45** | 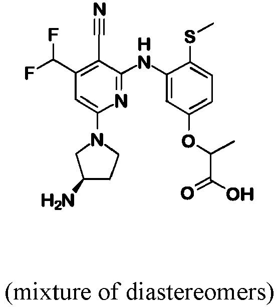 | 464 | 0.45 min (H) |
| **46** | 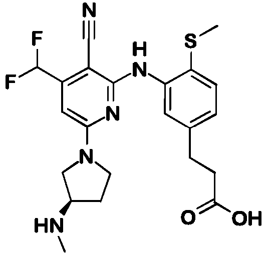 | 462 | 0.64 min (G) |
| **47** | 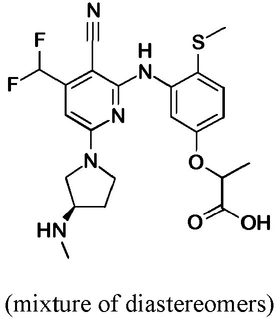 | 478 | 0.64 min (G) |
| **48** | 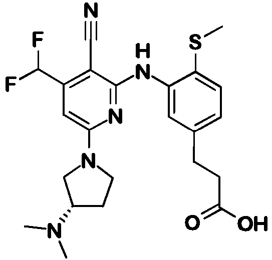 | 476 | 0.63 min (E) |
| **49** | 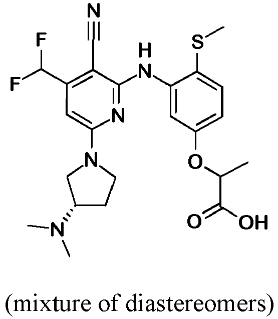 | 492 | 0.50 min (H) |

**Table 1b:**

| **Example #** | **Dichloro-pyridine reagent** | **Amine reagent** | **Anilin reagent** | procedures sequentially applied for synthesis |
|---|---|---|---|---|
| **5** | 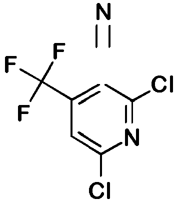 | 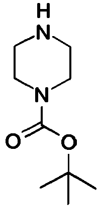 | 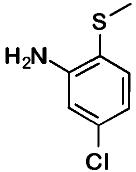 | A>B>C |
| **6** | 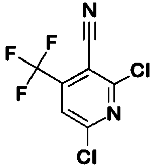 | 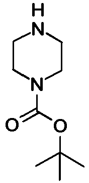 | 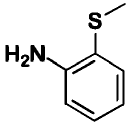 | A>B>C |
| **7** | 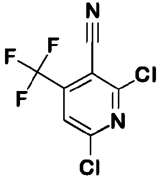 | 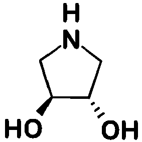 | 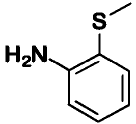 | A>B |
| **8** | 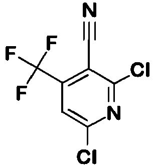 | methylamine (3 eq., no further base), reaction at 0 °C | 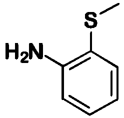 | A>B |
| **9** | 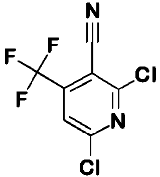 | 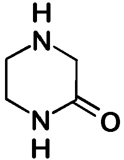 | 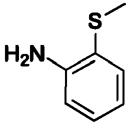 | A>B |
| **10** | 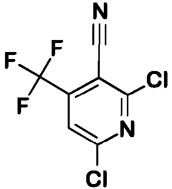 | 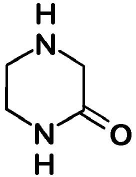 | 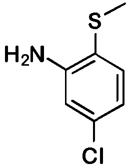 | A>B |
| **11** | 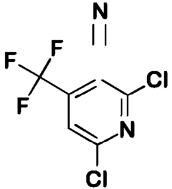 | 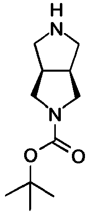 | 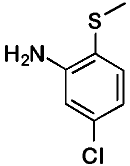 | A>B>C |
| **12** | 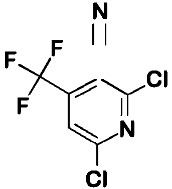 | 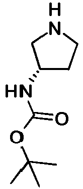 | 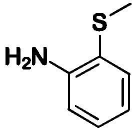 | A>B>C |
| **13** | 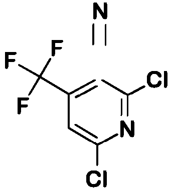 | 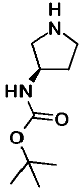 | 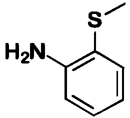 | A>B>C |
| **14** | 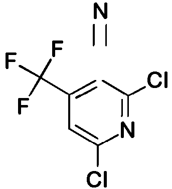 | 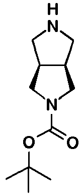 | 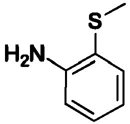 | A>B>C |
| **15** | 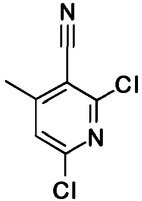 | 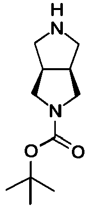 | 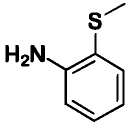 | A>B>C |
| **16** | 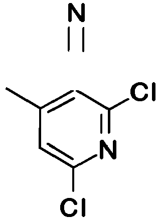 | 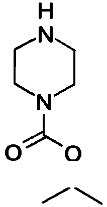 | 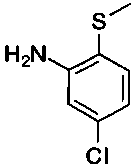 | A>B>C |
| **17** | 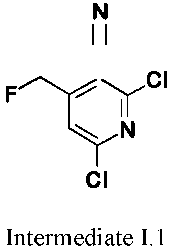 | 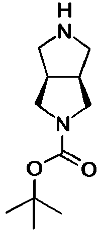 | 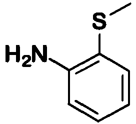 | A>B>C |
| **18** | 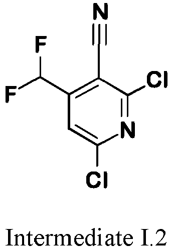 | 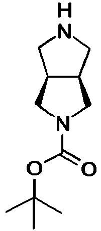 | 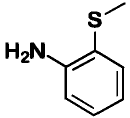 | A>B>C |
| **19** | 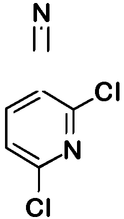 | 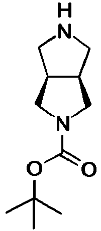 | 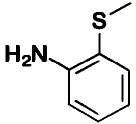 | A>B>C |
| **20** | 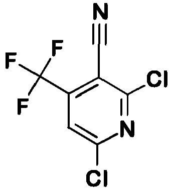 | 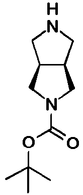 | 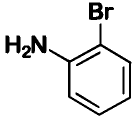 | A>B>C |
| **21** | 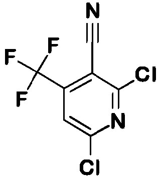 | 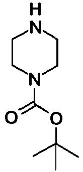 | 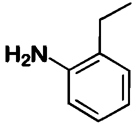 | A>B>C |
| **22** | 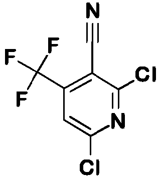 | 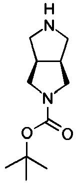 | 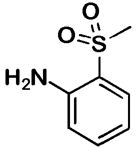 | A>B>C |
| **23** | 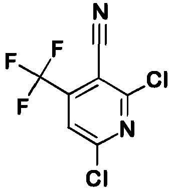 | 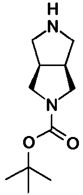 | 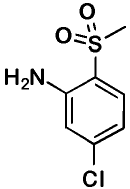 | A>B>C |
| **24** | 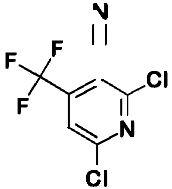 | 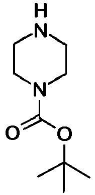 | 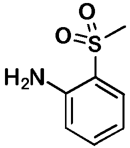 | A>B>C |
| **25** | 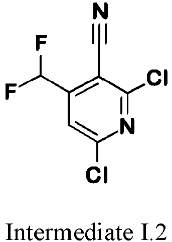 | 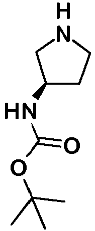 | 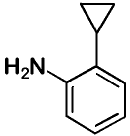 | A>B>C |
| **26** | 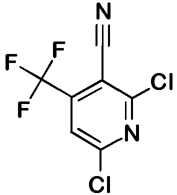 | 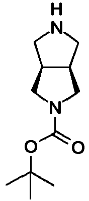 | 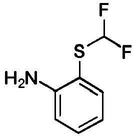 | A>B>C |
| **27** | 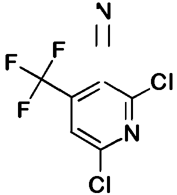 | 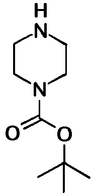 | 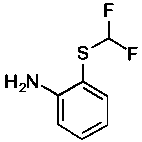 | A>B>C |
| **28** | 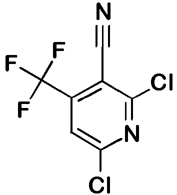 | 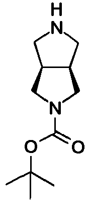 | 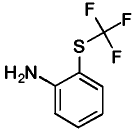 | A>B>C |
| **29** | 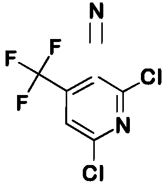 | 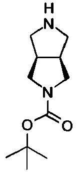 | 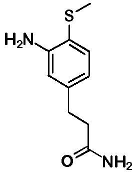 | A>B>C |
| **30** | 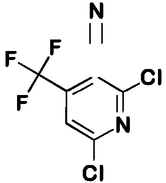 | 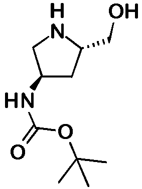 | 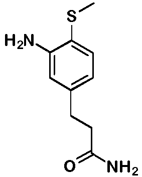 | A>B>C |
| **31** | 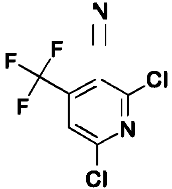 | 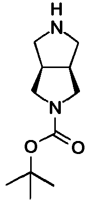 | 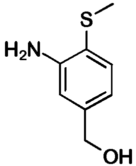 | A>B>C |
| **32** | 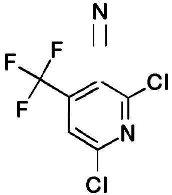 | 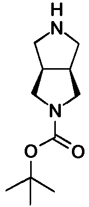 | 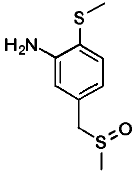 | A>B>C |
| **33** | 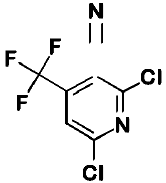 | 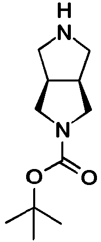 | 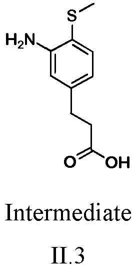 | A>B>C |
| **34** | 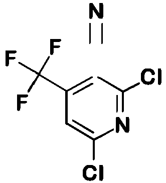 | 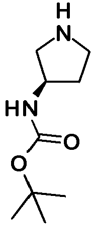 | 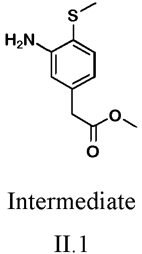 | A>B>C>D |
| **35** | 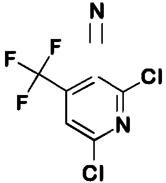 | 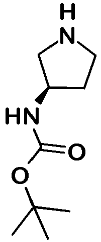 | 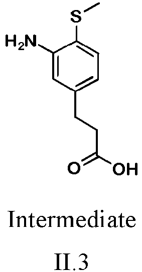 | A>B>C |
| **36** | 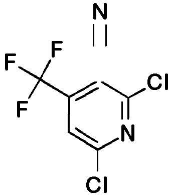 | 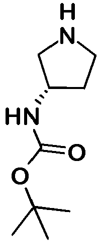 | 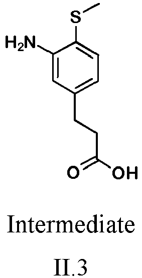 | A>B>C |
| **37** | 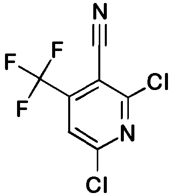 | 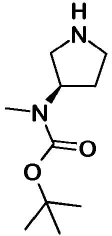 | 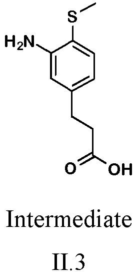 | A>B>C |
| **38** | 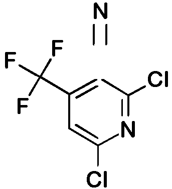 | 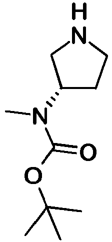 | 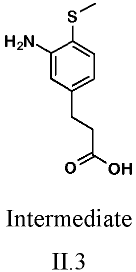 | A>B>C |
| **39** | 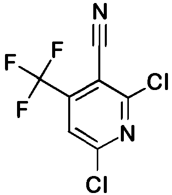 | 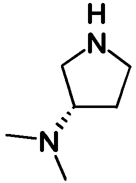 | 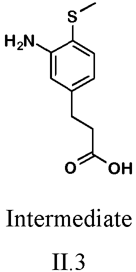 | A>B |
| **40** | 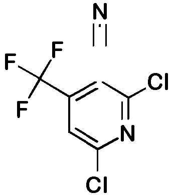 | 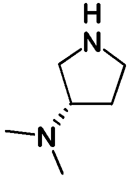 | 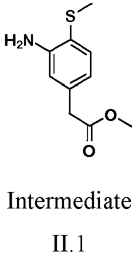 | A>B>D |
| **41** | 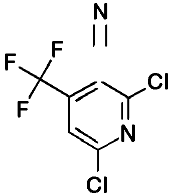 | 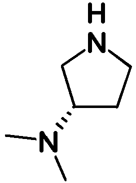 | 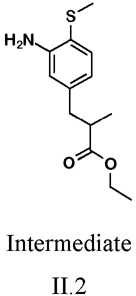 | A>B>D |
| **42** | 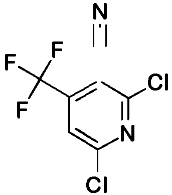 | 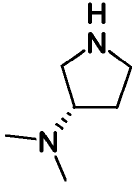 | 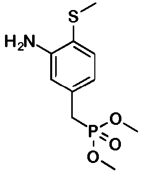 | A>B>LiBr cleavage: The phosphonic monoester was generated by stirring a solution of the phosphonic diester intermediate and LiBr (16 eq.) in ACN over night. |
| **43** | 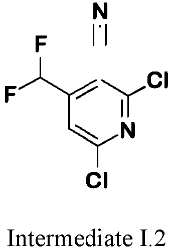 | 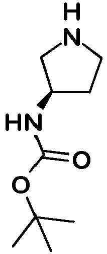 | 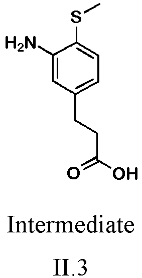 | A>B>C |
| **44** | 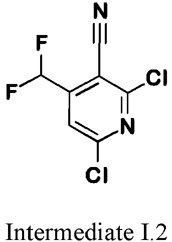 | 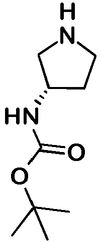 | 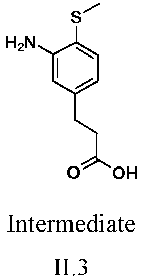 | A>B>C |
| **45** | 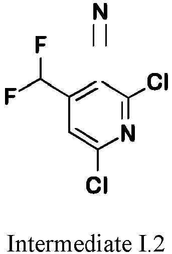 | 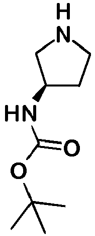 | 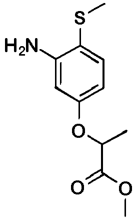 | A>B>C>D |
| **46** | 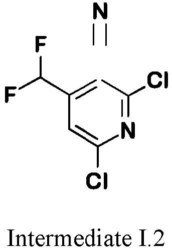 | 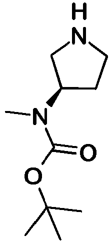 | 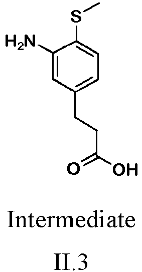 | A>B>C |
| **47** | 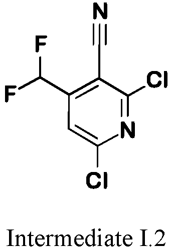 | 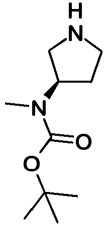 | 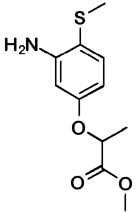 | A>B>C>D |
| **48** | 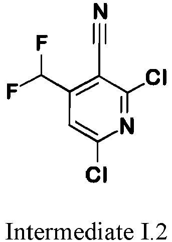 | 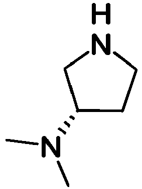 | 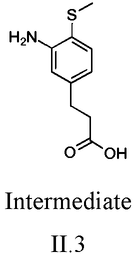 | A>B |
| **49** | 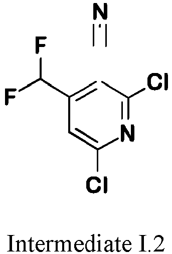 | 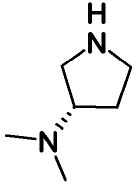 | 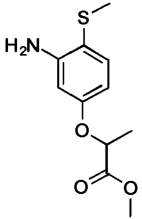 | A>B>D |

### Example 50

### ({[3-({6-[cis-octahydropyrrolo[3,4-c]pyrrol-2-yl]-3-cyano-4-(trifluoromethyl)pyridin-2-yl}amino)-4-methylsulfanyl)phenyl]methoxy}(methyl)phosphinic acid

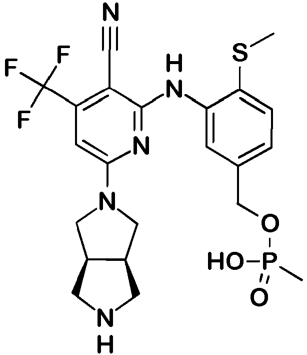

A mixture of **example 31** (100 mg; 0.222 mmol), methylphosphonic acid (21.4 g; 0.222 mmol), N,N'-dicyclohexylcarbodiimide (55.1 mg; 0.267 mmol) and a catalytic amount of DMAP in chloroform (10 ml) was gently refluxed over night. The mixture was evaporated to dryness and the crude product was purified by preparative RP-HPLC (column: C8; eluent: water/ACN/ammonia to yield the ammonium salt of the title compound (21 mg; 18%) as a colourless solid.
MS (ESI pos.+neg. Loop-Inj.) *m*/*z*: 528 [M + H]⁺
RT = 0.79 min (HPLC method D)

### Examples 51 and 52

Chiral separation of example 41 (3-[3-({3-cyano-6-[(3S)-3-(dimethylamino)pyrrolidin-1-yl]-4-(trifluoromethyl)pyridin-2-yl}amino)-4-methylsulfanyl)phenyl]-2-methylpropanoic acid)

The diastereomeric mixture **example 41** was separated applying chiral SFC:
Column: Lux^{®} Cellulose-2 10 x 250 mm 5 µm
Solvents: scCO2 70 %; MeOH+20mM NH3 30 %
Backpressure Regulator 120 bar
Temperature 40 °C
Flowrate 10 ml/min
Sample concentration 10 mg/ml
Sample solvent 100% MeOH
Injection volume 200 µl

Chiral purity was determined by analytic SFC applying the same column material and solvent.

### Example 51 (diastereomer eluting first; RT = 3.83 min):

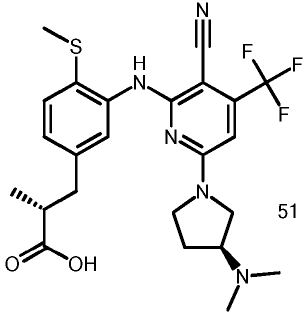

The absolute configuration of the methyl substituent in alpha position to the carboxylic acid was assigned arbitrarily, the pure diastereomer example 51 may therefore have the structure as shown or the structure shown for example 52.
MS (ESI pos.+neg. Loop-Inj.) *m*/*z*: 508 [M + H]⁺

### Example 52 (diastereomer eluting second; RT = 4.57 min):

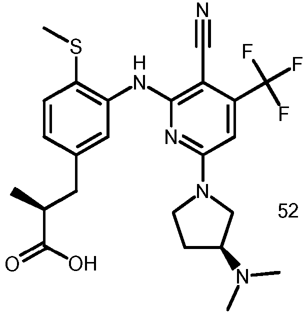

The absolute configuration of the methyl substituent in alpha position to the carboxylic acid was assigned arbitrarily, the pure diastereomer example 52 may therefore have the structure as shown or the structure shown for example 51.
MS (ESI pos.+neg. Loop-Inj.) *m*/*z*: 508 [M + H]⁺

### Biological Methods

### Assay A: Human KHK-C inhibition assay:

### Kinase activity of recombinant His-tagged KHK isoforms.

The enzymatic activity of recombinant human KHK-C was determined using the ADP-GLO^{™} Kinase Assay kit from Promega as described in the instructions. In brief, 1.25 µg/ml His tagged human KHK-A was incubated for 60 min at room temperature with 15 mM D-fructose. His-tagged human KHK-C (1 µg/ml) and His-tagged mouse KHK-C (0.625 µg/ml) were incubated for 60 min at room temperature with 400 µM D-fructose and 200 µM ATP. His-tagged rat KHK-C (0.5 µg/1.5 ml was incubated for 60 min at room temperature with 100 µM D-fructose and 200 µM ATP.

For all incubations the following assay buffer was used: 50mM HEPES pH 7.4, 4mM MgCl2, 20mM KCl, 0.01% Tween 20, 1mM DTT. The enzymatic reaction was stopped and developed using the ADP-GLO^{™} Kinase kit according manufacturers description and the results were analyzed using the luminescence signal determined by a multiplate reader from EnVision. Signals from samples with enzyme and substrates alone were reported as 100 % and signals from samples with enzyme alone were reported as 0 %.

### Assay B: Inhibition of fructose-1-phosphate generation in HepG2 cells:

### Quantitative determination of fructose-1-phosphate in HepG2 cells.

HepG2 cells (B.B Knowles, Wistar Institute) were incubated with test compound or solvent (DMSO) in medium (EMEM, 10 mM NEAA, 8 mM glutamine, 10 % FCS) for 30 min at 37°C under 5 % CO₂. D-fructose was added to a final concentration of 15 mM and cells were incubated for further 60 min under the same conditions. Cells were put on ice washed with phosphate buffered saline and lysed in 10 mM ammonium acetate. Cell protein was precipitated with acetonitrile and an aliquot of the supernatant was analyzed for fructose-1-phosphate using the RapidFire-MS/MS (RIAS) technology. Fructose-6-phosphate (0.1 µM) in the samples was used as internal standard for the quantification.

The following Table shows IC₅₀ values of example compounds determined using assay A:

| **cpd #** | **KHK inhibition assay (Assay A) IC₅₀ (nM)** |
|---|---|
| 1 | 7.5 |
| 2 | 18.7 |
| 3 | 24.6 |
| 4 | 12.8 |
| 5 | 23.2 |
| 6 | 10.9 |
| 7 | 20.3 |
| 8 | 17.6 |
| 9 | 33.3 |
| 10 | 72.1 |
| 11 | 1.3 |
| 12 | 10.1 |
| 13 | 5.5 |
| 14 | 4.2 |
| 15 | 5.8 |
| 16 | 21.5 |
| 17 | 12.1 |
| 18 | 5.0 |
| 19 | 19.4 |
| 20 | 63.0 |
| 21 | 176.2 |
| 22 | 6.5 |
| 23 | 7.4 |
| 24 | 91.0 |
| 25 | 3.7 |
| 26 | 1.6 |
| 27 | 10.8 |
| 28 | 31.1 |
| 29 | 2.0 |
| 30 | 3.2 |
| 31 | 1.5 |
| 32 | 5.0 |
| 33 | 0.6 |
| 34 | 6.1 |
| 35 | 5.0 |
| 36 | 5.7 |
| 37 | 4.8 |
| 38 | 7.4 |
| 39 | 13.0 |
| 40 | 8.0 |
| 41 | 26.7 |
| 42 | 16.9 |
| 43 | 17.9 |
| 44 | 12.8 |
| 45 | 4.1 |
| 46 | 4.7 |
| 47 | 2.8 |
| 48 | 9.4 |
| 49 | 9.3 |
| 50 | 2.3 |
| 51 | 39.5 |
| 52 | 26.8 |

The following Table shows IC₅₀ values of example compounds determined using assay B:

| **Example cpd #** | **HepG2 assay (Assay B) IC₅₀ (nM)** |
|---|---|
| 3 | 125 |
| 4 | 123 |
| 14 | 124 |
| 18 | 63 |
| 22 | 194 |
| 26 | 107 |
| 29 | 90 |
| 33 | 168 |
| 39 | 156 |
| 48 | 64 |
| 51 | 284 |
| 52 | 565 |

Comparison of the example compounds with the prior art compound 6-[(3S,4S)-3,4-Dihydroxy-1-pyrrolidinyl]-2-[(3R)-3-hydroxy-3-methyl-1-pyrrolidinyl]-4-(trifluoromethyl)-3-pyridinecarbonitrile (CAS Registry Number: 2711012-28-9).

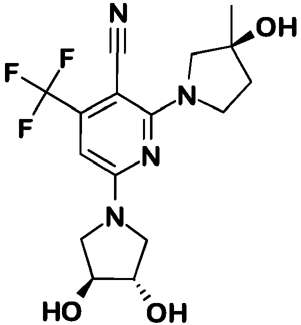

This prior art compound (Huard et al., J. Med. Chem. 2017, 60, 7835-7849) has been synthesized and then tested in assay A under the same conditions as the example compounds of this invention. The IC₅₀ value of this compound has been determined to be 187 nM.

## Claims

1. A compound having formula (I) or a salt thereof 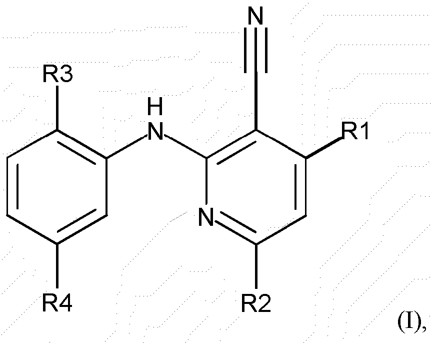 wherein:
**R1** is 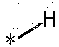 (H), 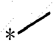 (Me), 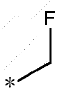 (CH₂F), 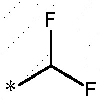 (CHF₂) or 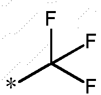 (CF₃),
**R2** is, 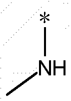 (methylamino), 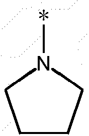 (1-pyrrolidinyl), substituted with 1 or two substituents selected from NH₂ such as in 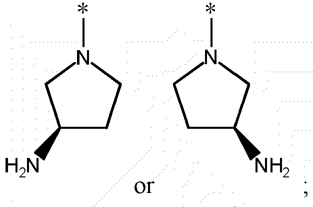
NHMe such as in 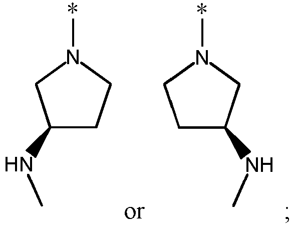 and
NMe₂ such as in 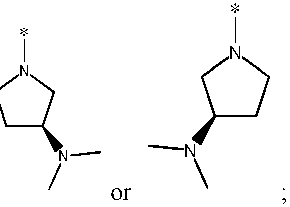 hydroxymethyl such as in 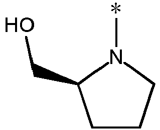
hydroxy such as in 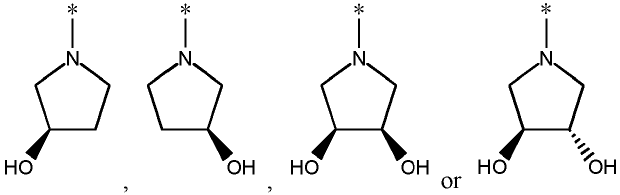 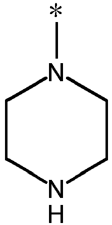 (1-piperazinyl); 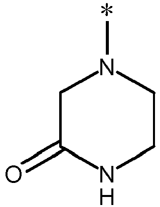 (3-keto-1-piperazinyl); or 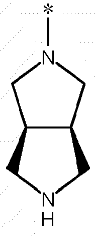 (cis-octahydropyrrolo[3,4-c]pyrrole-2-yl);
**R3** is, 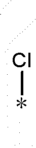 (Cl), 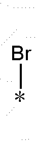 (Br), 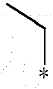 (Et), 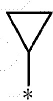 (cyclopropyl), 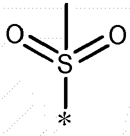 (SO₂Me), or 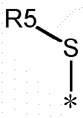 (S-R5), wherein R5 is Me or Et optionally substituted with up to three F
such as in 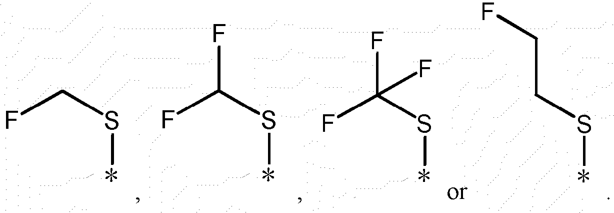
**R4** is 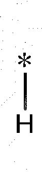 (H), 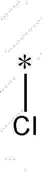 (Cl), 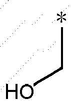 (CH₂OH), 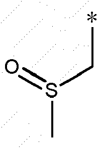 (CH₂-S(O)-CH₃₎, 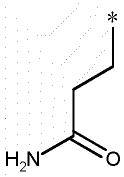 (CH₂-CH₂-CONH₂); 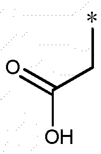 (CH₂COOH), 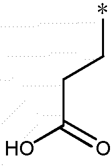 (CH₂-CH₂-COOH), 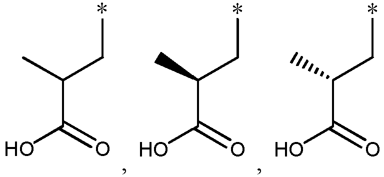 (CH₂-CH(Me)-COOH), 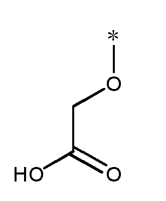 (O-CH₂-COOH); 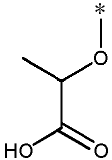 (O-CH(Me)-COOH); 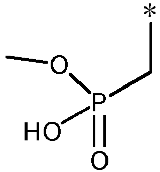 (CH₂-P(O)(OH)-OMe); or 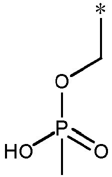 (CH₂-O-P(O)(OH)-Mₑ).

2. The compound of claim 1, wherein **R1** is CHF₂ or CF₃.

3. The compound of claim 1, wherein **R2** is 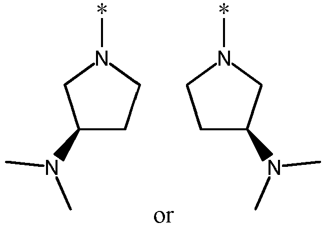 (3-dimethylamino-pyrrolidin-1-yl).

4. The compound of claim 1, wherein **R3** is 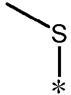 (SMe), 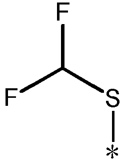 (SCHF₂) or cyclopropyl.

5. The compound of claim 4 or a salt thereof, wherein **R3** is SO₂Me.

6. The compound of claim 1 or a salt thereof, wherein **R4** is 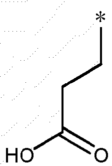 (CH₂-CH₂-COOH) or 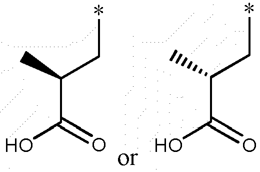 (CH₂-CH(Me)-COOH).

7. The compound of claim 1 or a salt thereof selected from the group of formula (I) compounds 1-52
| **Example #** | **Structure** |
|---|---|
| **1** | 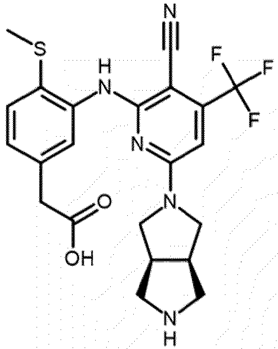 |
| **2** | 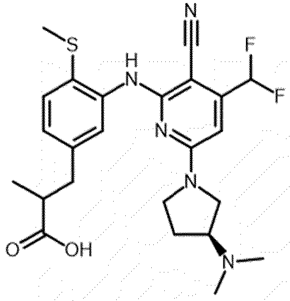 |
| **3** | 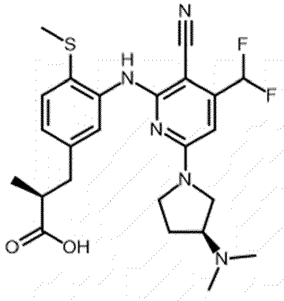 |
| **4** | 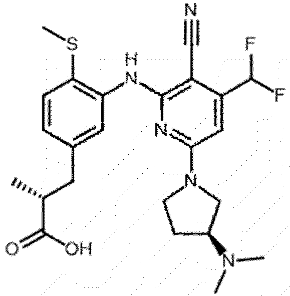 |
| **5** | 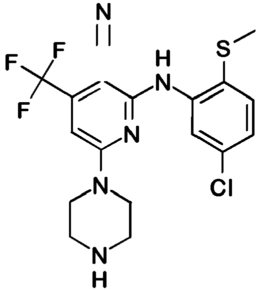 |
| **6** | 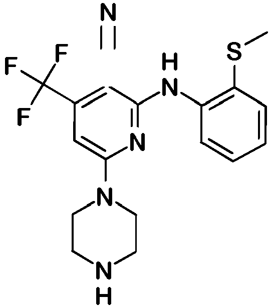 |
| **7** | 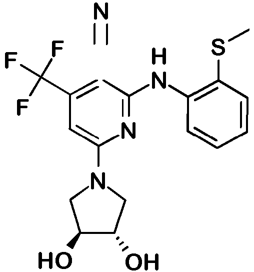 |
| **8** | 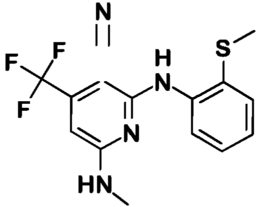 |
| **9** | 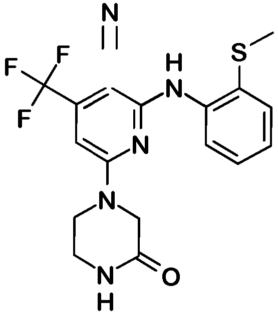 |
| **10** | 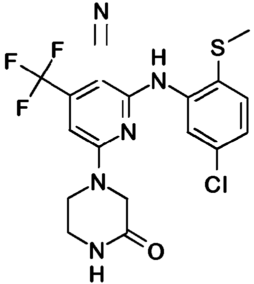 |
| **11** | 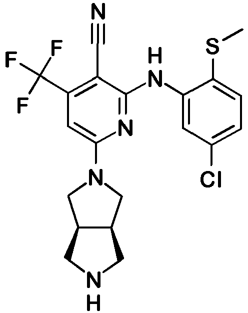 |
| **12** | 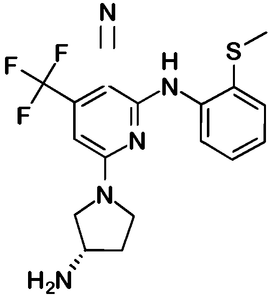 |
| **13** | 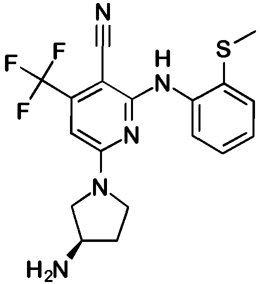 |
| **14** | 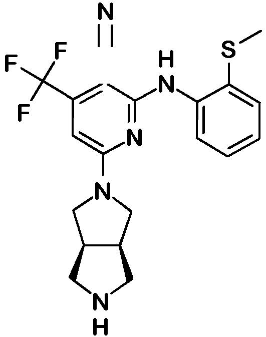 |
| **15** | 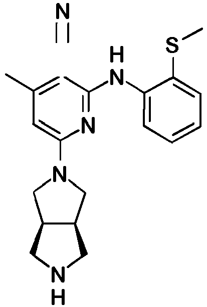 |
| **16** | 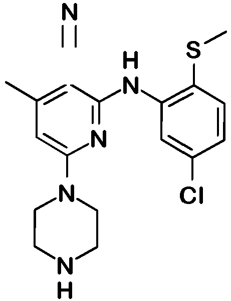 |
| **17** | 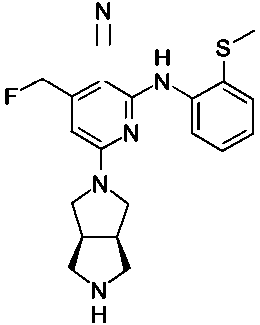 |
| **18** | 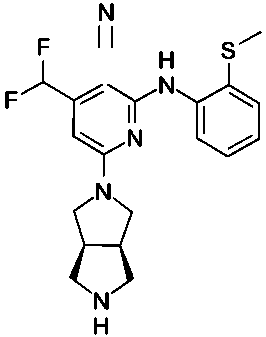 |
| **19** | 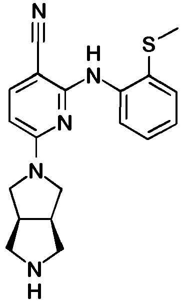 |
| **20** | 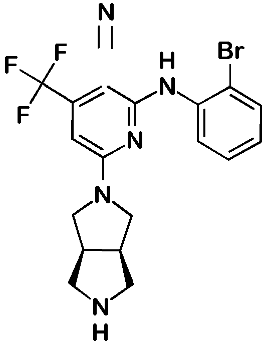 |
| **21** | 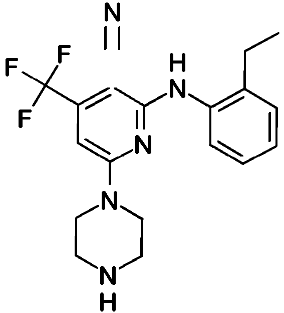 |
| **22** | 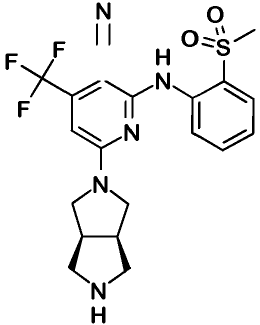 |
| **23** | 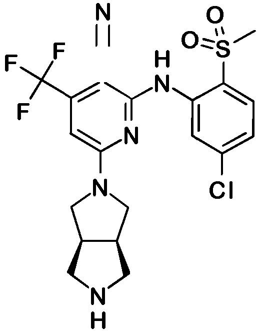 |
| **24** | 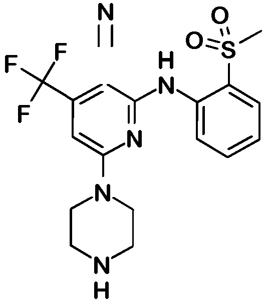 |
| **25** | 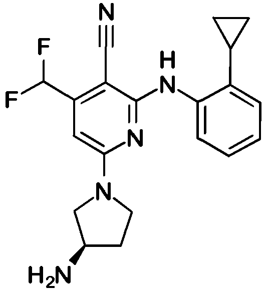 |
| **26** | 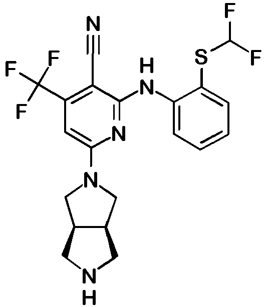 |
| **27** | 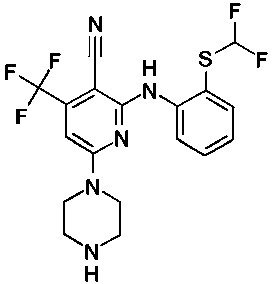 |
| **28** | 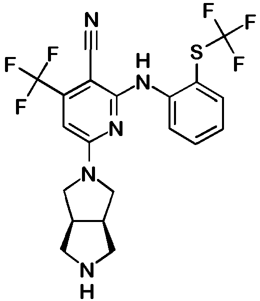 |
| **29** | 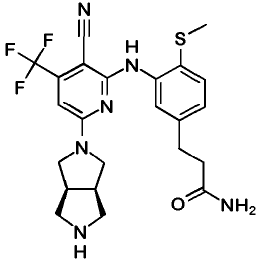 |
| **30** | 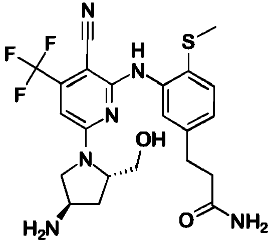 |
| **31** | 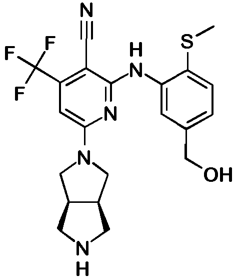 |
| **32** | 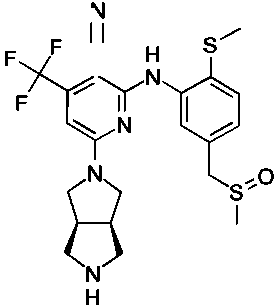 |
| **33** | 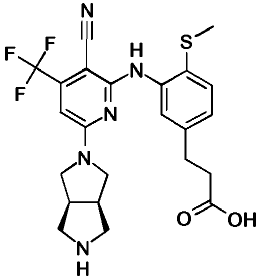 |
| **34** | 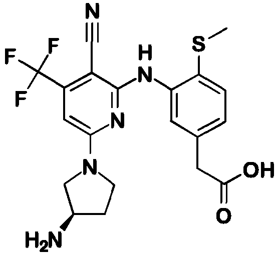 |
| **35** | 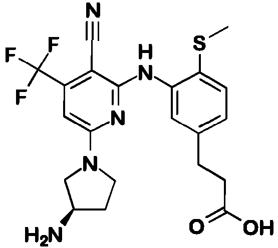 |
| **36** | 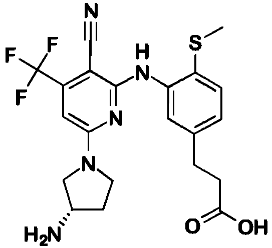 |
| **37** | 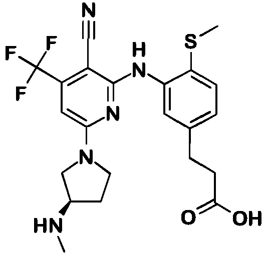 |
| **38** | 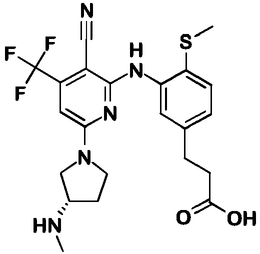 |
| **39** | 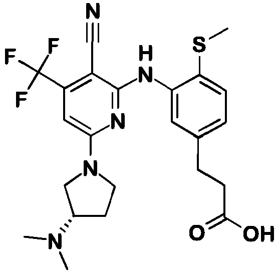 |
| **40** | 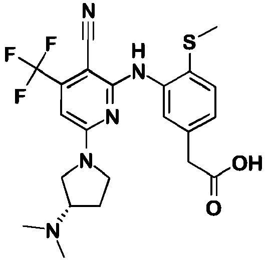 |
| **41** | 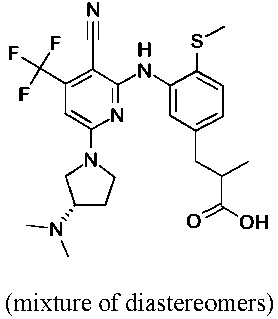 |
| **42** | 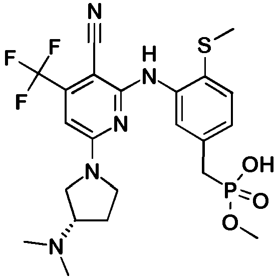 |
| **43** | 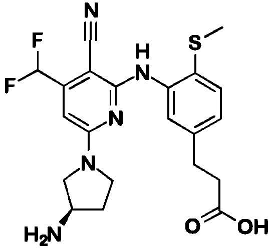 |
| **44** | 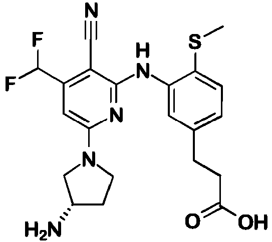 |
| **45** | 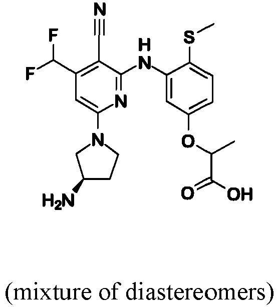 |
| **46** | 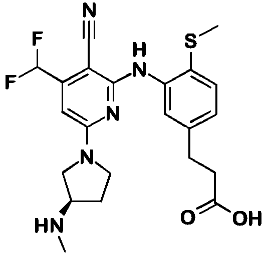 |
| **47** | 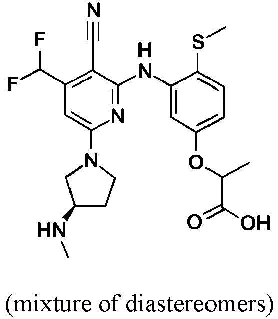 |
| **48** | 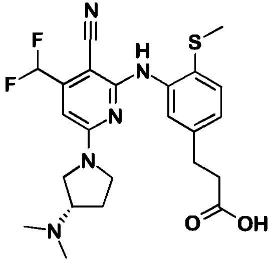 |
| **49** | 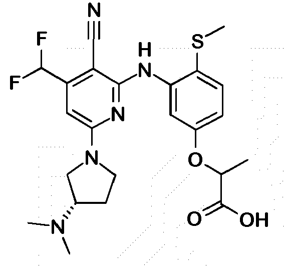 (mixture of diastereomers) |
| **50** | 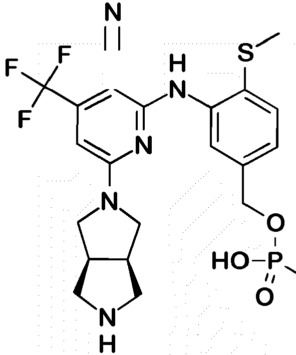 |
| **51** | 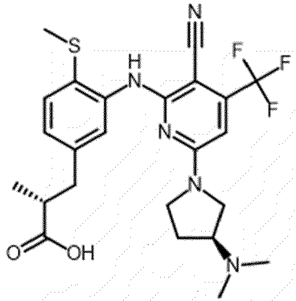 |
| **52** | 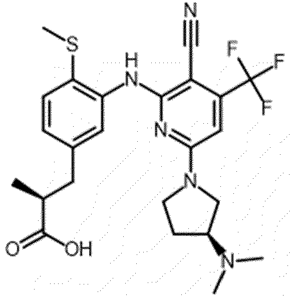 |

8. The salt of a compound according to any one of claims 1 to 7 for use as a medicament.

9. A medicament comprising a compound or salt thereof according to any one of claims 1 to 7.

10. A method for the preparation of the compound of claim 1, comprising reacting
chloropyridine compounds of general formula (II) 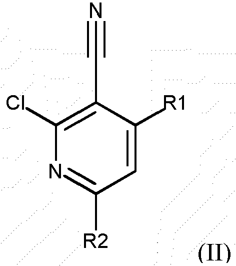
with anilins of general formula (III) 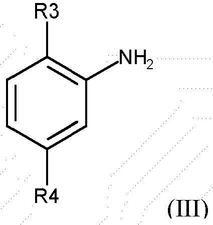
under cross-coupling conditions applying a palladium catalyst such as. XPhos Pd G2
and a base such as cesium carbonate in a solvent such as dioxane.

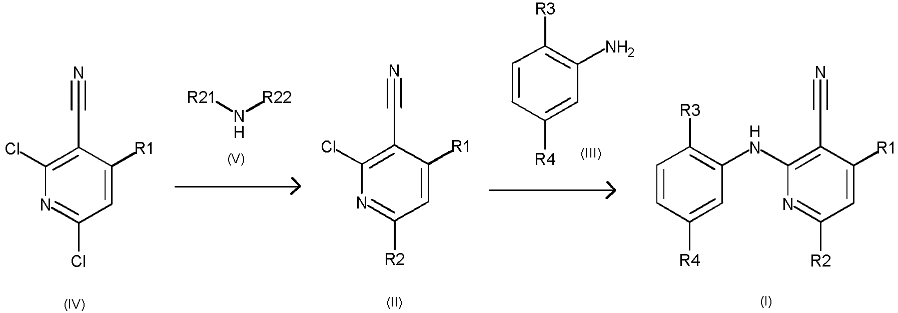

11. The method according to claim 10, **characterized in that** the compounds of general formula (II) are prepared from dichloropyridines of general formula (IV) 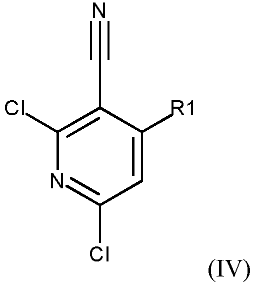 through reaction with an approprioate primary or secondary amine (V) in the presence of a base such as N,N-diisopropylethylamine in a solvent such as ethanol.

12. A pharmaceutical composition containing at least one compound according to one or more of the claims 1 to 7 or a pharmaceutically acceptable salt thereof together with one or more pharmaceutically acceptable carrier.

13. The pharmaceutical composition according to claim 12 comprising a therapeutically effective amount of a compound according to any one of claims 1 to 7 in the range from 0.1 to 90 wt.-% of the composition as a whole, preferably in the range from 0.5 to 50 wt.-% of the composition as a whole, or a pharmaceutically acceptable salt thereof,

14. A compound according to one or more of claims 1 to 7, or a pharmaceutically acceptable salt thereof or a pharmaceutical composition according to claim 12 for use in the treatment or prevention of non-alcoholic fatty liver disease (NAFLD), non-alcoholic steatohepatitis (NASH) and type II diabetes (T2DM).

15. A compound according to any one of claims 1 to 7, or a salt thereof, for use according to claim 14, wherein said compound is administered before, after or together with at least one other pharmaceutically active substance.

## Patentansprüche

1. Verbindung mit der Formel (I) oder deren Salz 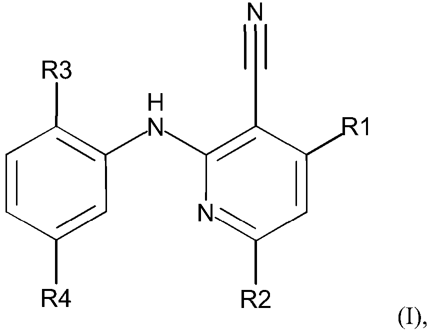 wobei:
**R1** ist: 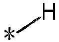 (H), 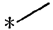 (Me), 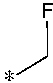 (CH₂F), 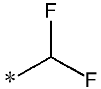 (CHF₂) oder 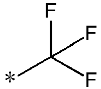 (CF₃),
**R2** ist: 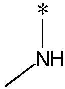 (Methylamino), 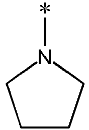 (1-Pyrrolidinyl), substituiert mit 1 oder zwei Substituenten, ausgewählt aus NH₂, wie in 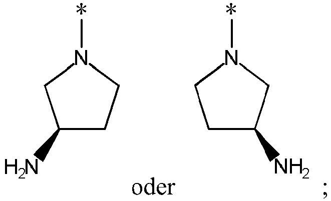
NHMe wie in 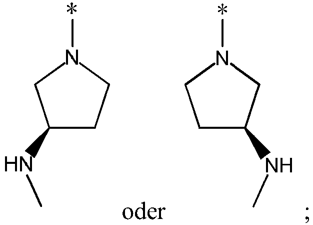 und
NMe₂ wie in 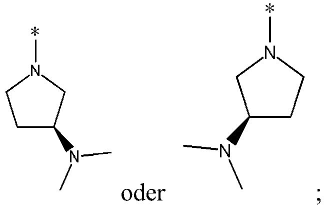
Hydroxymethyl wie in 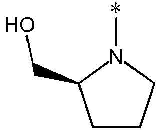
Hydroxy wie in 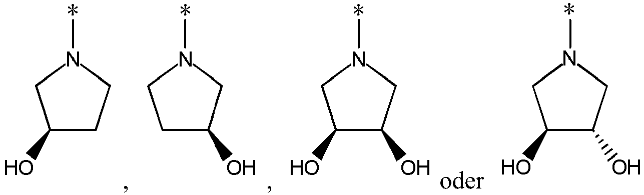 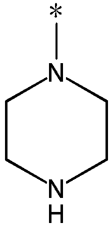 (1-Piperazinyl); 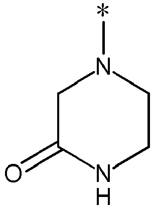 (3-Keto-1-piperazinyl); oder 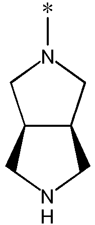 (cis-Octahydropyrrolo[3,4-c]pyrrol-2-yl);
**R3** ist: 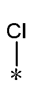 (Cl), 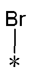 (Br), 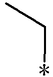 (Et), 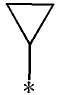 (Cyclopropyl), 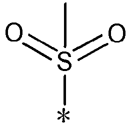 (SO₂Me); oder 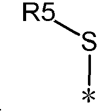 (S-R5), wobei R5 Me oder Et darstellt, gegebenenfalls substituiert mit bis
zu drei F wie in 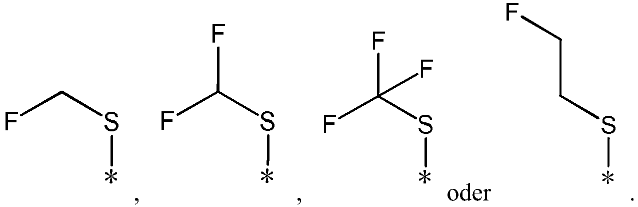
**R4** ist: 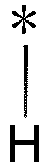 (H), 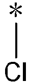 (Cl), 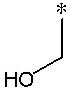 (CH₂OH), 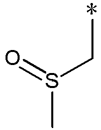 (CH₂-S(O)-CH_{3),} 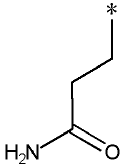 (CH₂-CH₂-CONH₂); 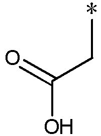 (CH₂COOH), 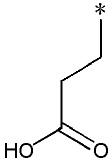 (CH₂-CH₂-COOH), 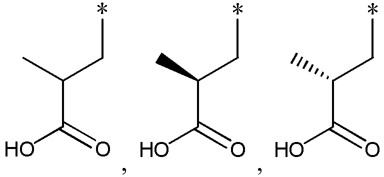 (CH₂-CH(Me)-COOH), 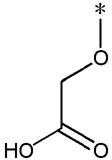 (O-CH₂-COOH); 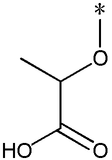 (O-CH(Me)-COOH); 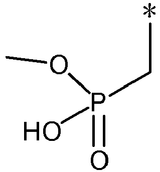 (CH₂-P(O)(OH)-OMe); oder 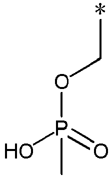 (CH₂-O-P(O)(OH)-Me).

2. Verbindung nach Anspruch 1, wobei **R1** CHF₂ oder CF₃ darstellt.

3. Verbindung nach Anspruch 1, wobei **R2** 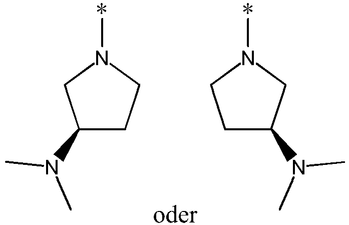 (3-Dimethylamino-pyrrolidin-1-yl) darstellt.

4. Verbindung nach Anspruch 1, wobei **R3** 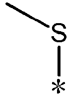 (SMe), 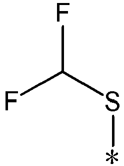 (SCHF₂) oder Cyclopropyl darstellt.

5. Verbindung nach Anspruch 4 oder deren Salz, wobei **R3** SO₂Me darstellt.

6. Verbindung nach Anspruch 1 oder deren Salz, wobei **R4** 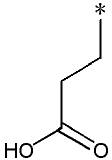 (CH₂-CH₂-COOH) oder 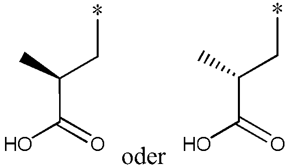 (CH₂-CH(Me)-COOH) darstellt.

7. Verbindung nach Anspruch 1 oder deren Salz, ausgewählt aus der Gruppe der Verbindungen der Formel (I) 1-52
| **Beispiel #** | **Struktur** |
|---|---|
| **1** | 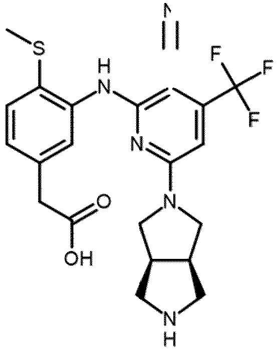 |
| **2** | 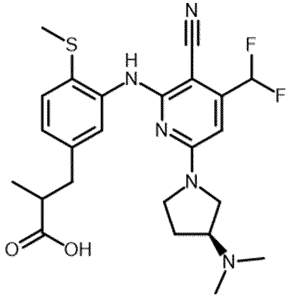 |
| **3** | 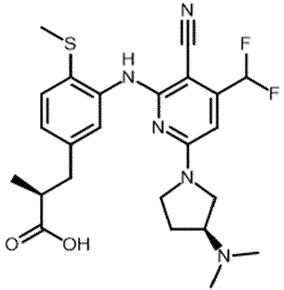 |
| **4** | 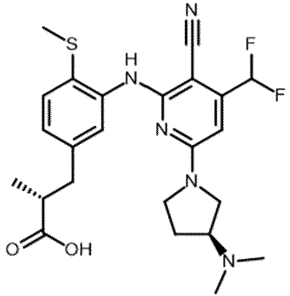 |
| **5** | 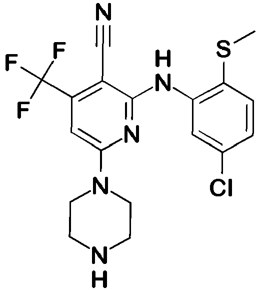 |
| **6** | 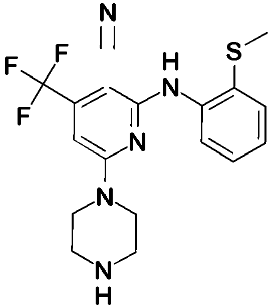 |
| **7** | 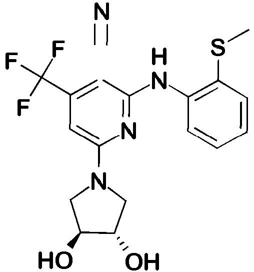 |
| **8** | 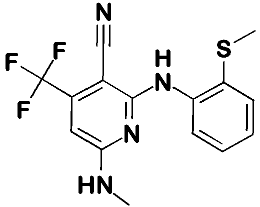 |
| **9** | 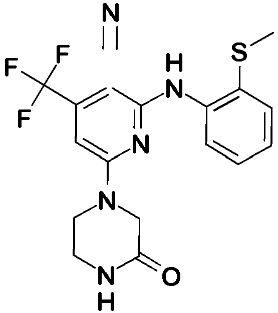 |
| **10** | 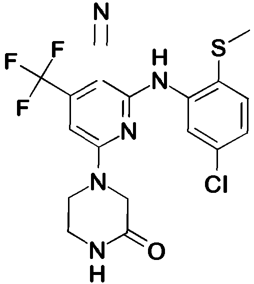 |
| **11** | 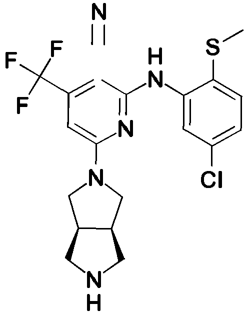 |
| **12** | 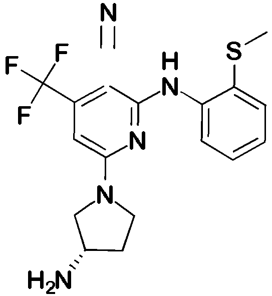 |
| **13** | 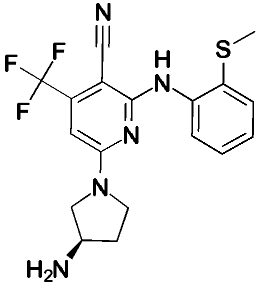 |
| **14** | 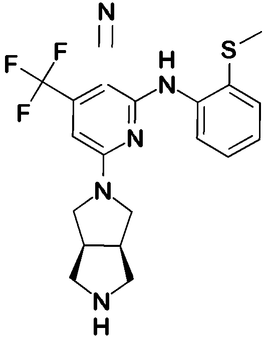 |
| **15** | 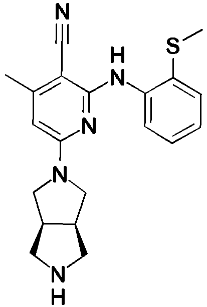 |
| **16** | 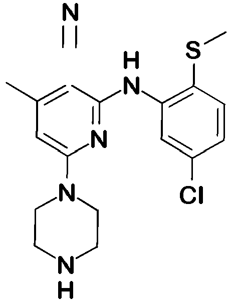 |
| **17** | 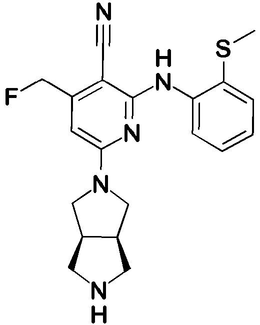 |
| **18** | 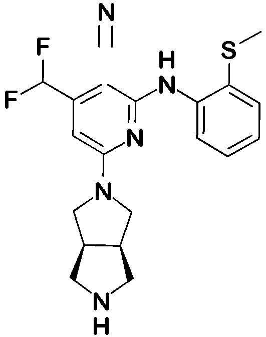 |
| **19** | 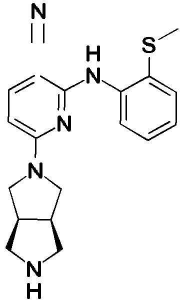 |
| **20** | 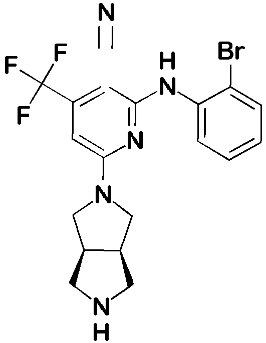 |
| **21** | 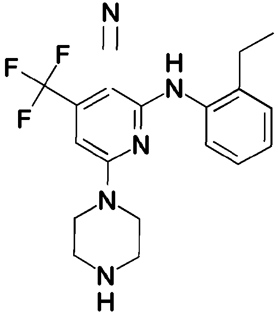 |
| **22** | 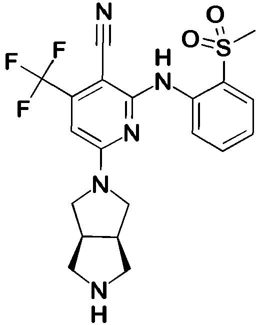 |
| **23** | 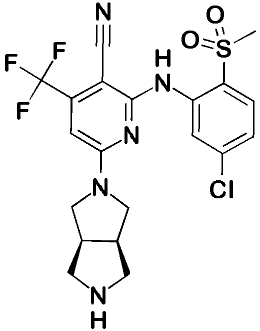 |
| **24** | 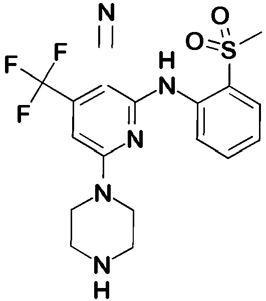 |
| **25** | 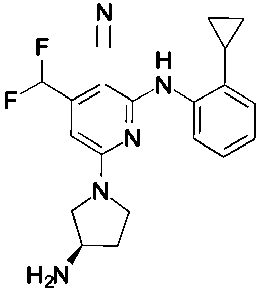 |
| **26** | 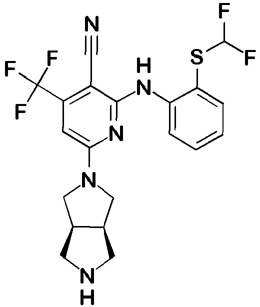 |
| **27** | 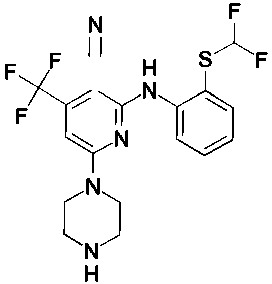 |
| **28** | 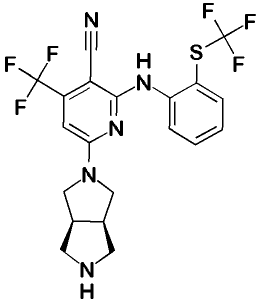 |
| **29** | 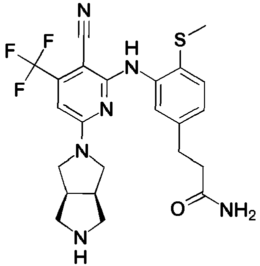 |
| **30** | 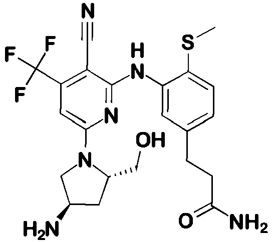 |
| **31** | 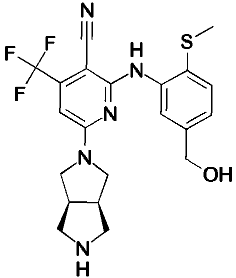 |
| **32** | 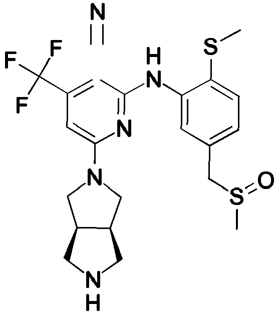 |
| **33** | 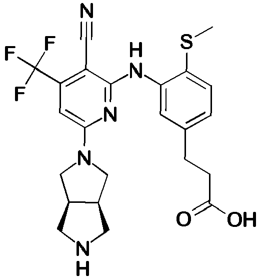 |
| **34** | 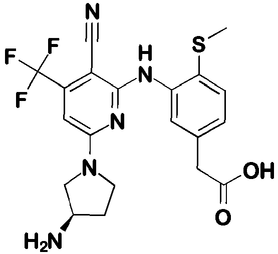 |
| 35 | 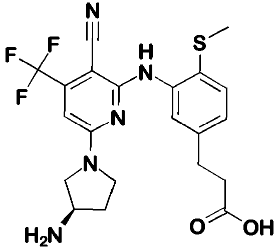 |
| 36 | 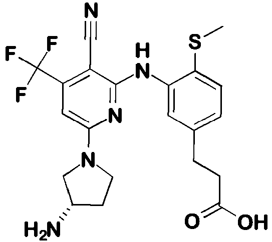 |
| 37 | 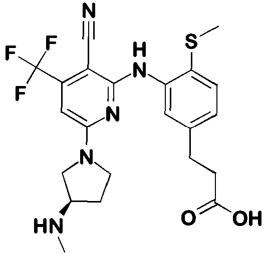 |
| 38 | 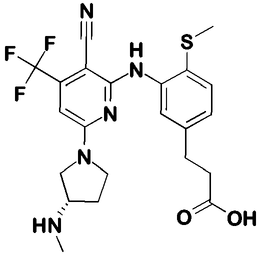 |
| **39** | 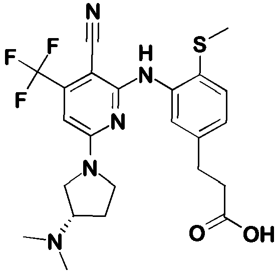 |
| **40** | 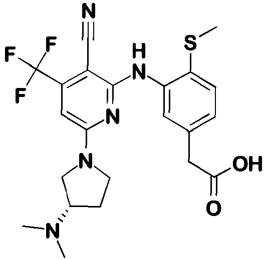 |
| **41** | 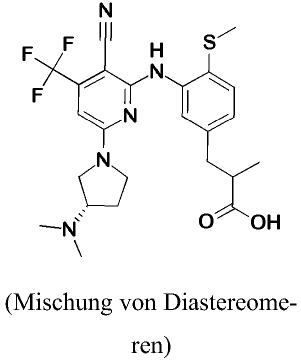 |
| **42** | 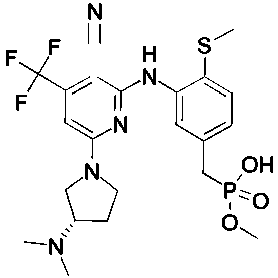 |
| **43** | 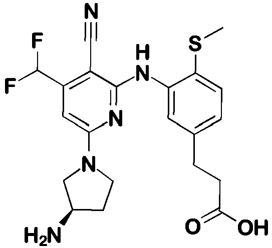 |
| **44** | 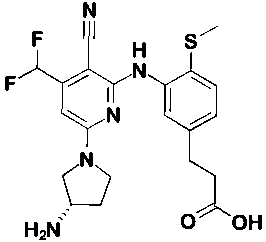 |
| **45** | 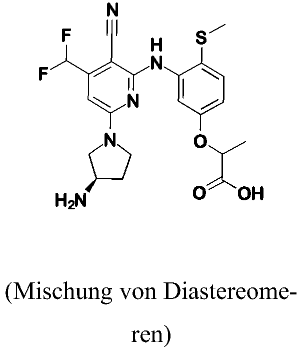 |
| **46** | 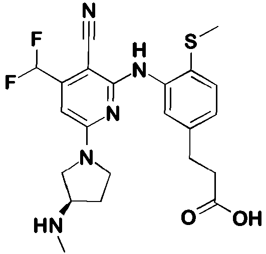 |
| **47** | 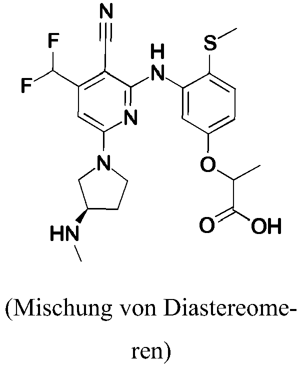 |
| **48** | 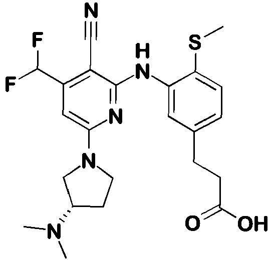 |
| **49** | 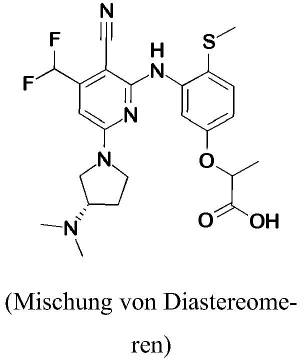 |
| **50** | 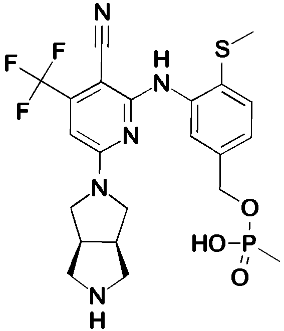 |
| **51** | 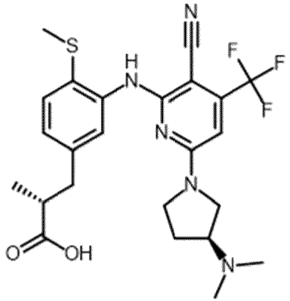 |
| **52** | 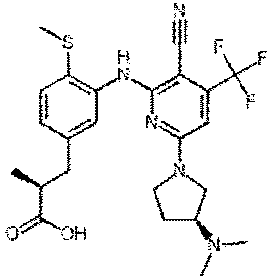 |

8. Salz einer Verbindung nach irgendeinem der Ansprüche 1 bis 7 zur Verwendung als Medikament.

9. Medikament, umfassend eine Verbindung oder deren Salz nach irgendeinem der Ansprüche 1 bis 7.

10. Verfahren zur Herstellung der Verbindung von Anspruch 1, umfassend die Umsetzung von Chlorpyridinverbindungen der allgemeinen Formel (II) 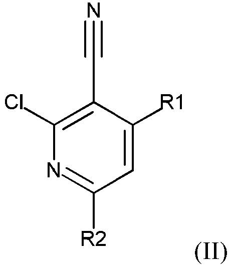
mit Anilinen der allgemeinen Formel (III) 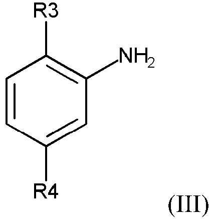
unter Kreuzkopplungsbedingungen, unter Verwendung eines Palladiumkatalysators wie XPhos Pd G2 und einer Base wie Cäsiumcarbonat in einem Lösungsmittel wie Dioxan.

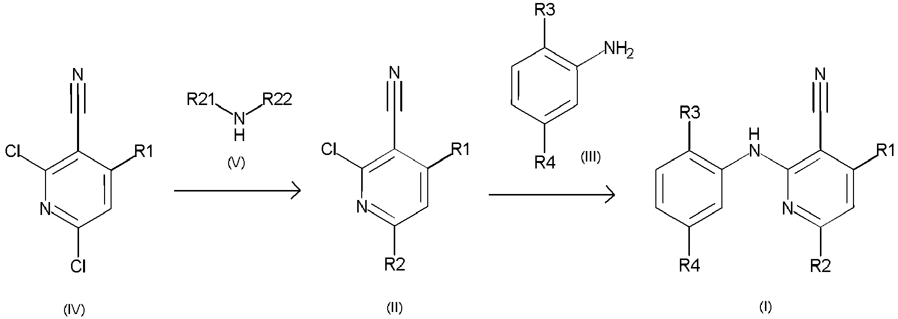

11. Verfahren nach Anspruch 10, **dadurch gekennzeichnet, dass** die Verbindungen der allgemeinen Formel (II) aus Dichlorpyridinen der allgemeinen Formel (IV) hergestellt werden 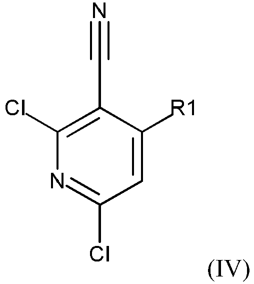 durch Umsetzung mit einem geeigneten primären oder sekundärem Amin (V) in Gegenwart einer Base wie N,N-Diisopropylethylamin in einem Lösungsmittel wie Ethanol.

12. Pharmazeutische Zusammensetzung, enthaltend mindestens eine Verbindung nach einem oder mehreren der Ansprüche 1 bis 7 oder deren pharmazeutisch akzeptables Salz, zusammen mit einem oder mehreren pharmazeutisch akzeptablen Trägern.

13. Pharmazeutische Zusammensetzung nach Anspruch 12, umfassend eine therapeutisch wirksame Menge einer Verbindung nach irgendeinem der Ansprüche 1 bis 7 im Bereich von 0,1 to 90 Gew.-% der Zusammensetzung als Ganzem, bevorzugt im Bereich von 0,5 to 50 Gew.-% der Zusammensetzung als Ganzem, oder deren pharmazeutisch akzeptables Salz.

14. Verbindung nach irgendeinem der Ansprüche 1 bis 7 oder deren pharmazeutisch akzeptables Salz oder eine pharmazeutische Zusammensetzung nach Anspruch 12 zur Verwendung in der Behandlung oder Vorbeugung von nicht-alkoholischer Fettlebererkrankung (NAFLD), nicht-alkoholischer Steatohepatitis (NASH) und Diabetes Typ II (T2DM).

15. Verbindung nach irgendeinem der Ansprüche 1 bis 7 oder deren Salz zur Verwendung nach Anspruch 14, wobei die Verbindung vor, nach oder zusammen mit mindestens einer anderen pharmazeutisch wirksamen Substanz verabreicht wird.

## Revendications

1. Composé de formule (I) ou sel de celui-ci 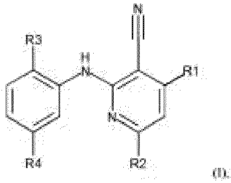 dans lequel :
**R1** est 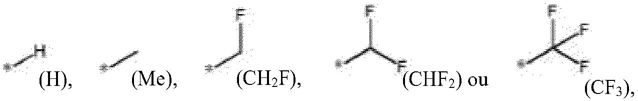
**R2** est, 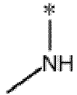 (méthylamino) , 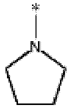 (1-pyrrolidinyle),
substitué avec 1 ou deux substituants sélectionnés parmi NH₂ tel que dans 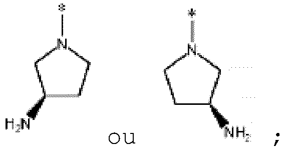
NHMe tel que dans 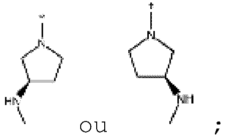 et
NMe₂ tel que dans 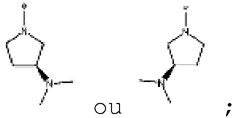 hydroxyméthyle tel que dans 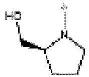 hydroxy tel que dans 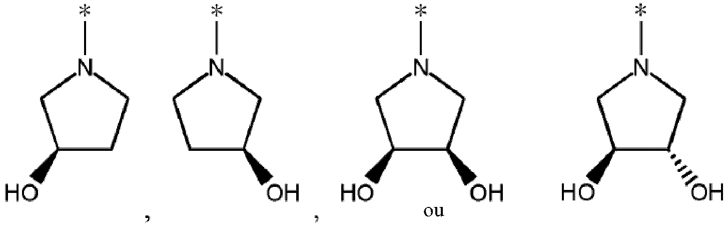 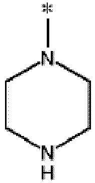 (1-pipérazinyle) ; 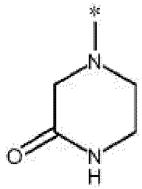 (3-céto-1-pipérazinyle) ; ou 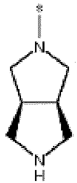 (cis-octahydropyrrolo[3,4-c]pyrrole-2-yle) ;
**R3** est, (Cl), 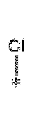 (Br), 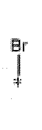 (Et) 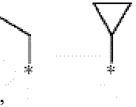 (cyclopropyle), 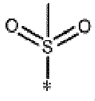 (SO₂Me), ou 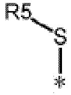 (S-R5), dans lequel R5 est Me ou Et facultativement substitué avec jusqu'à trois F tels que 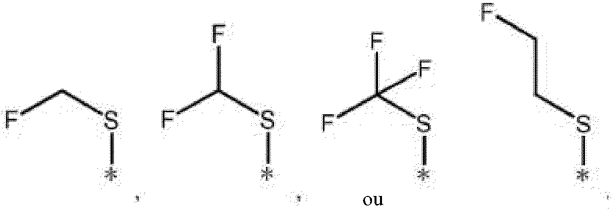 dans
**R4** est 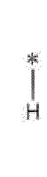 (H), 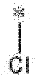 (Cl), 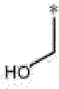 (CH₂OH), 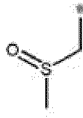 (CH₂-S(O)-CH₃), 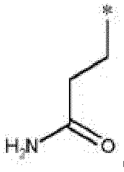 (CH₂-CH₂-CONH₂) ; 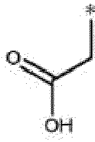 (CH₂COOH), 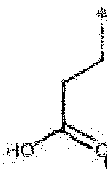 (CH₂-CH₂-COOH), 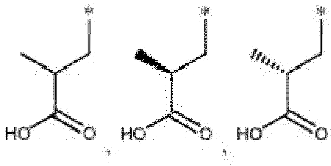 (CH₂-CH(Me)-COOH), 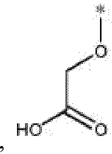 (O-CH₂-COOH) ; 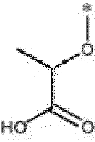 (O-CH(Me)-COOH) ; 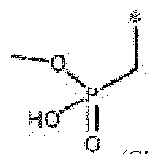 (CH₂- P(O)(OH)-OMe) ; ou 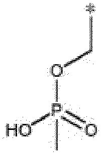 (CH₂-O-P(O)(OH)-Me).

2. Composé selon la revendication 1, dans lequel R1 est CHF₂ ou CF₃.

3. Composé selon la revendication 1, dans lequel **R2** est 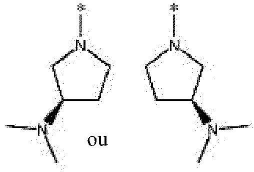 (3-diméthylamino-pyrrolidin-1-yle).

4. Composé selon la revendication 1, dans lequel **R3** est 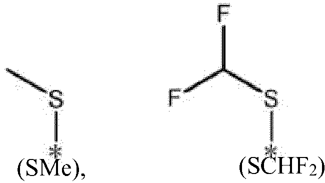 ou cyclopropyle.

5. Composé selon la revendication 4 ou sel de celui-ci, dans lequel **R3** est SO₂Me.

6. Composé selon la revendication 1 ou sel de celui-ci, dans lequel **R4** est 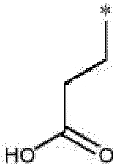 (CH₂-CH₂- COOH) ou 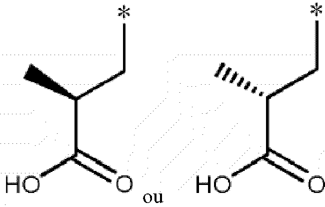 (CH₂-CH(Me)-COOH).

7. Composé selon la revendication 1 ou sel de celui-ci, sélectionné dans le groupe de composés 1 à 52 de formule (I)
| **Exemple #** | **Structure** |
|---|---|
| **1** | 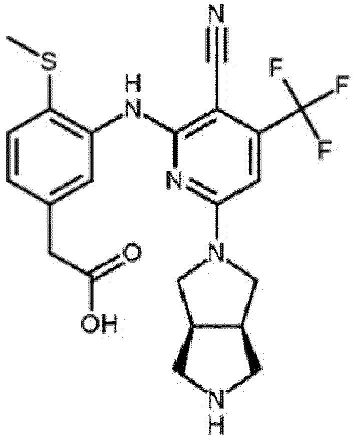 |
| **2** | 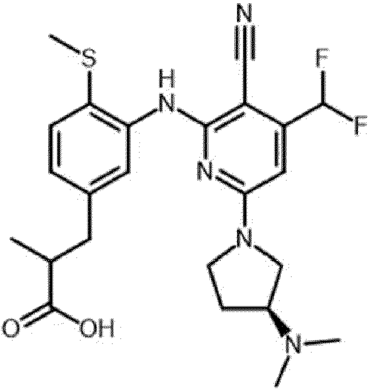 |
| **3** | 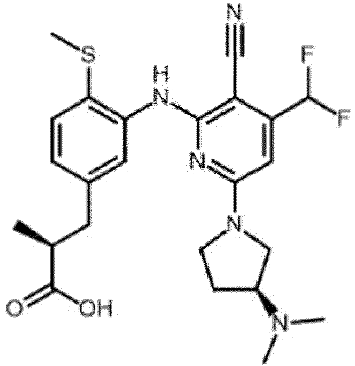 |
| **4** | 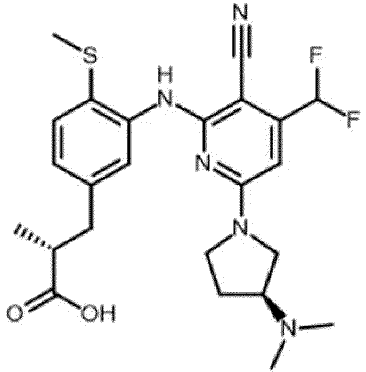 |
| **5** | 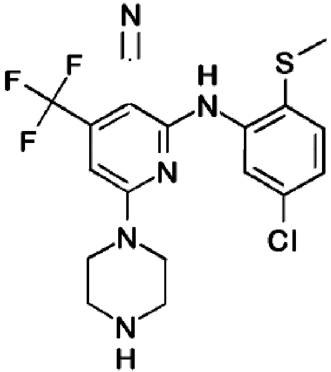 |
| **6** | 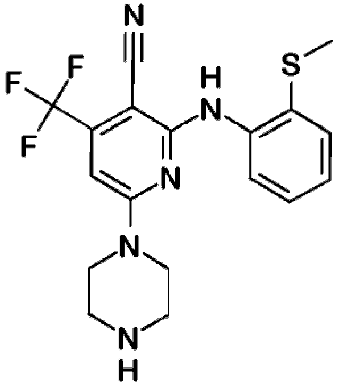 |
| **7** | 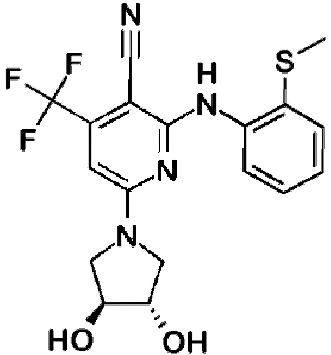 |
| **8** | 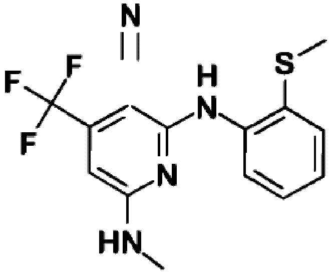 |
| **9** | 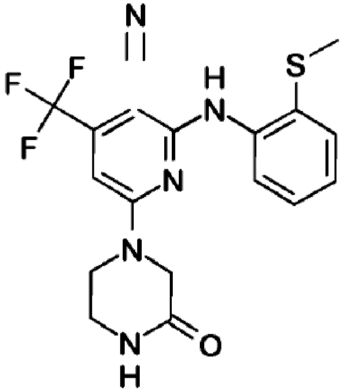 |
| **10** | 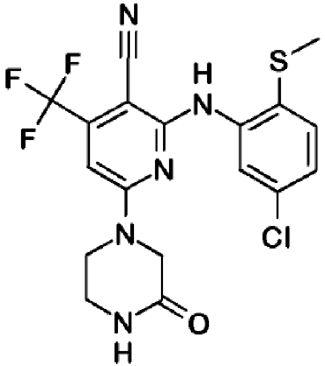 |
| **11** | 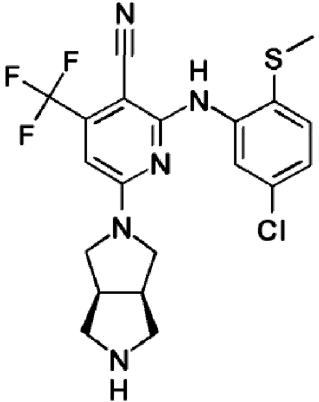 |
| **12** | 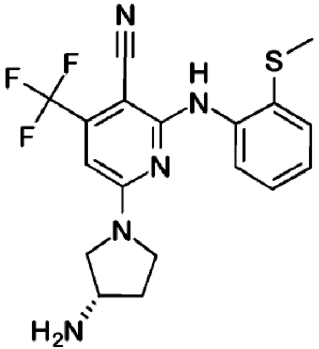 |
| **13** | 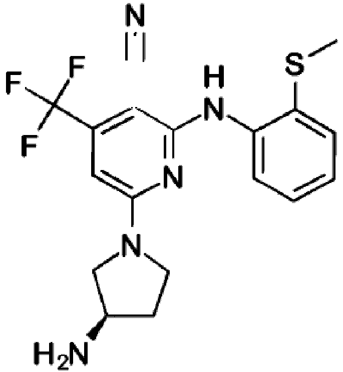 |
| **14** | 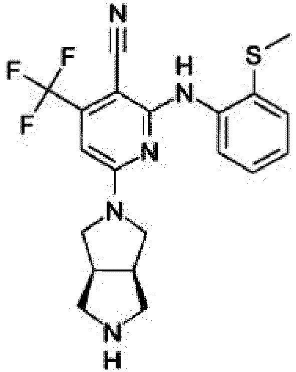 |
| **15** | 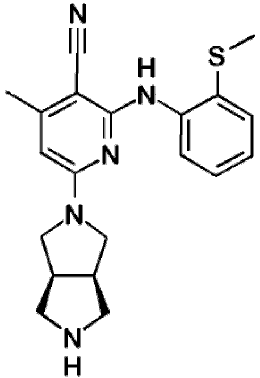 |
| **16** | 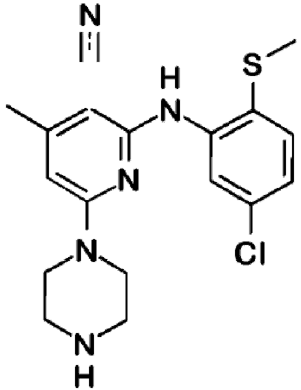 |
| **17** | 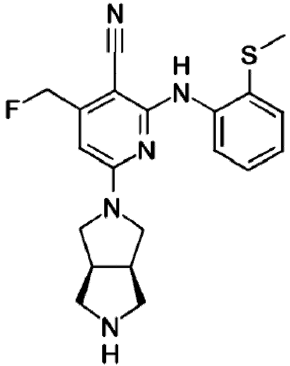 |
| **18** | 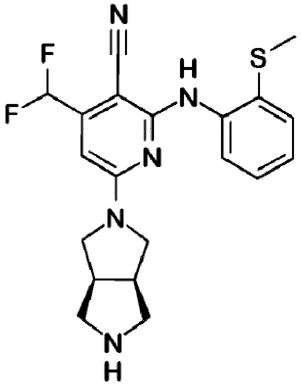 |
| **19** | 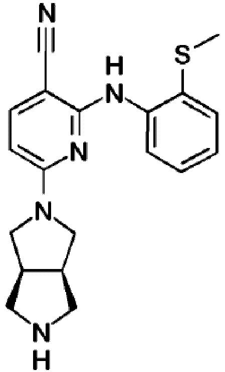 |
| **20** | 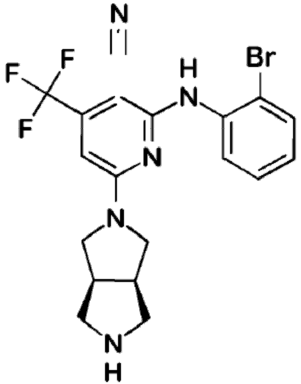 |
| **21** | 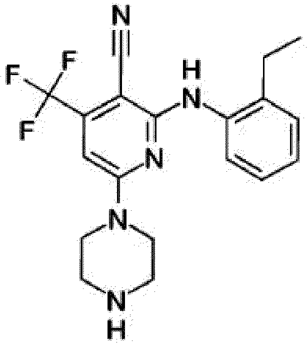 |
| **22** | 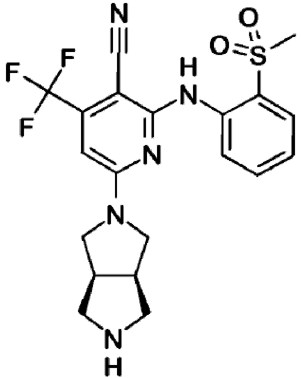 |
| **23** | 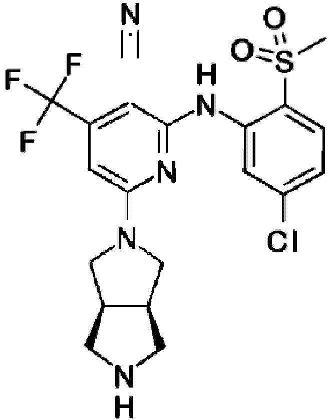 |
| **24** | 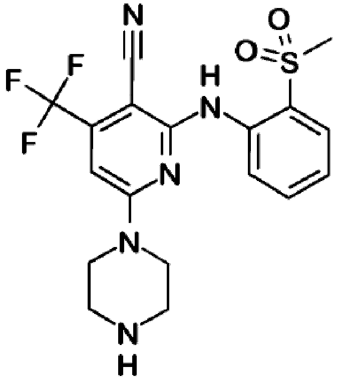 |
| **25** | 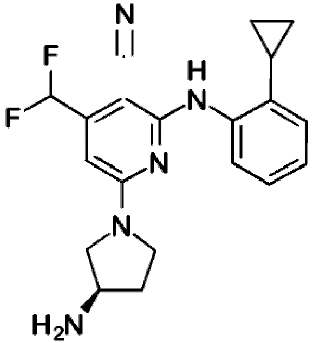 |
| **26** | 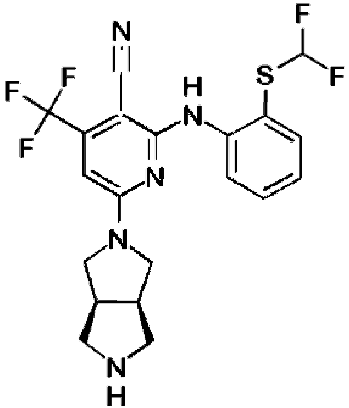 |
| **27** | 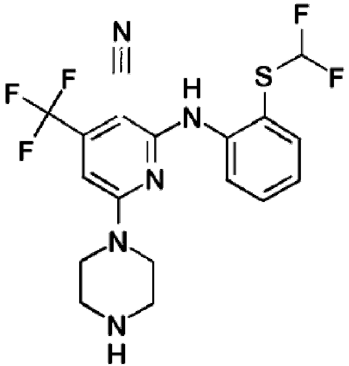 |
| **28** | 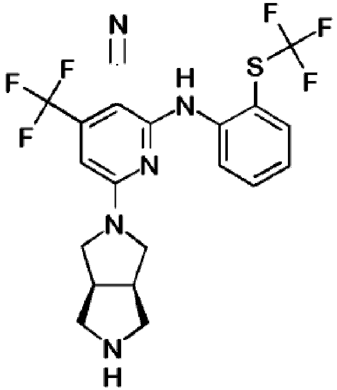 |
| **29** | 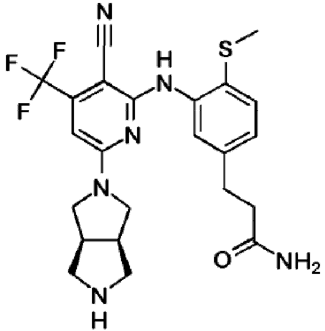 |
| **30** | 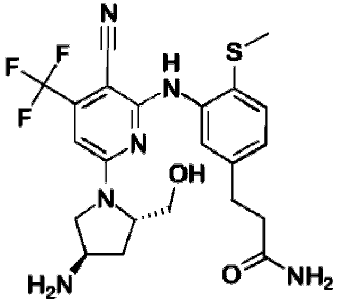 |
| **31** | 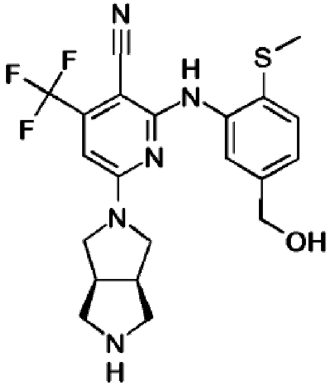 |
| **32** | 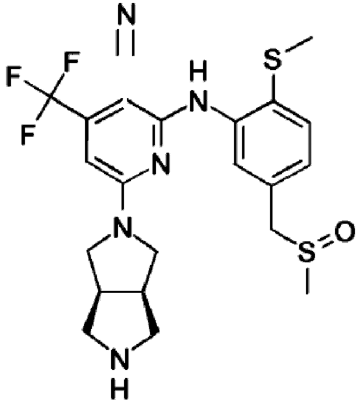 |
| **33** | 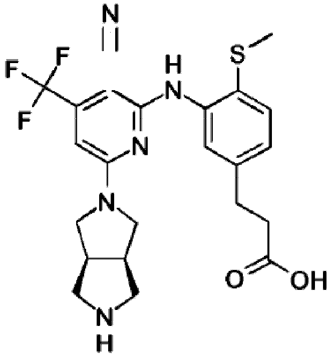 |
| **34** | 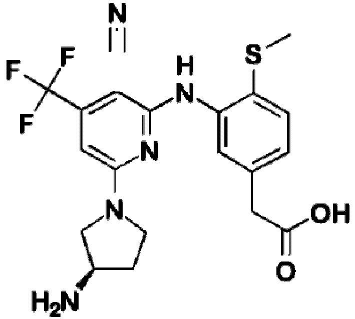 |
| **35** | 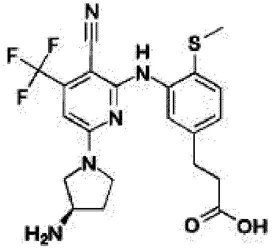 |
| **36** | 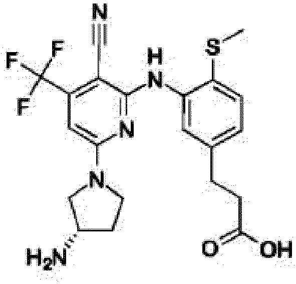 |
| **37** | 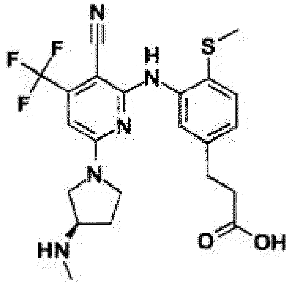 |
| **38** | 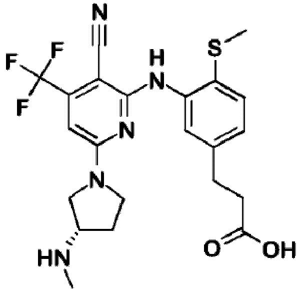 |
| **39** | 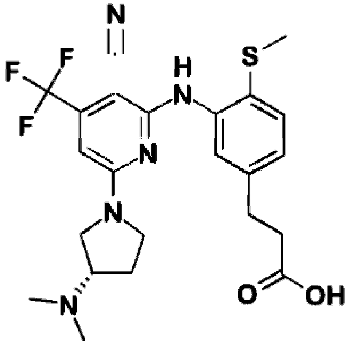 |
| **40** | 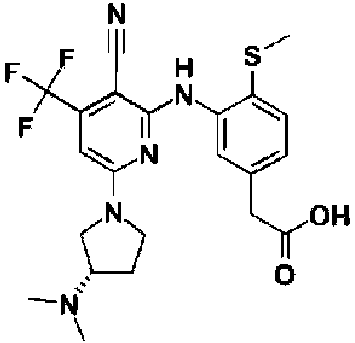 |
| **41** | 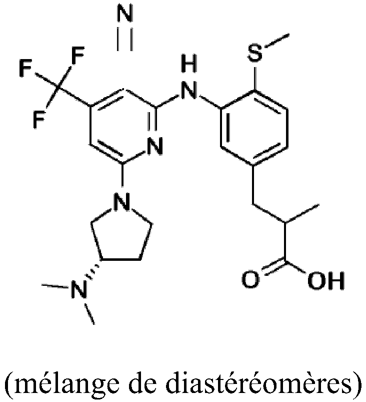 |
| **42** | 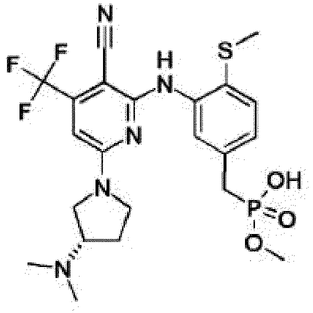 |
| **43** | 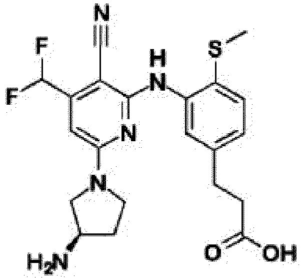 |
| **44** | 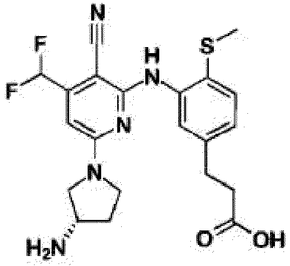 |
| **45** | 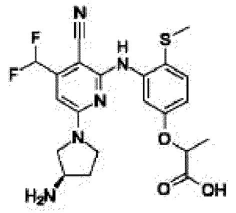 (mélange de diastéréomères) |
| **46** | 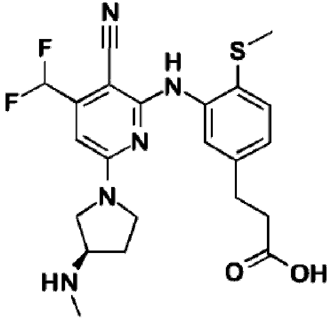 |
| **47** | 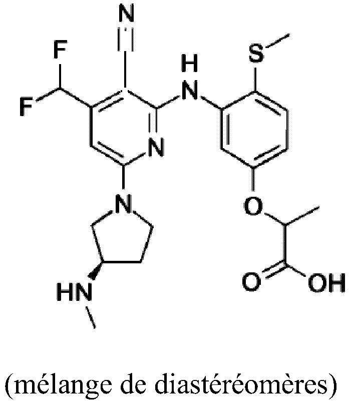 |
| **48** | 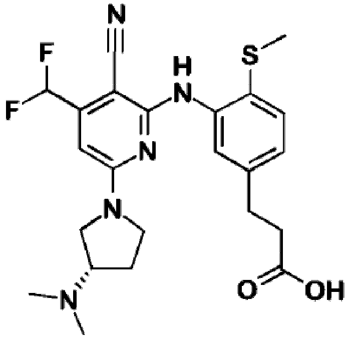 |
| **49** | 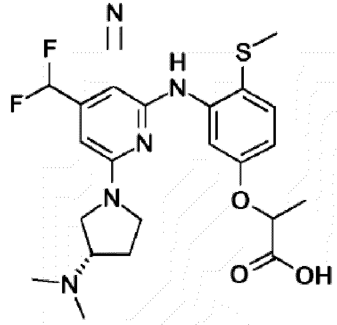 (mélange de diastéréomères) |
| **50** | 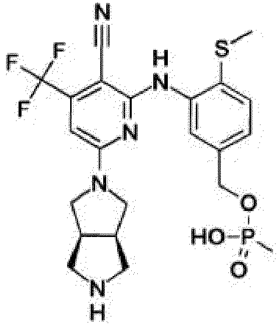 |
| **51** | 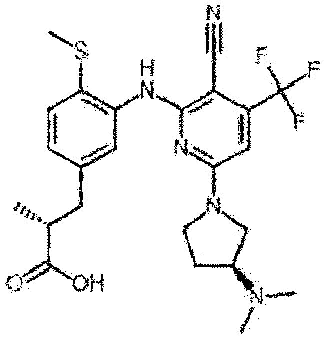 |
| **52** | 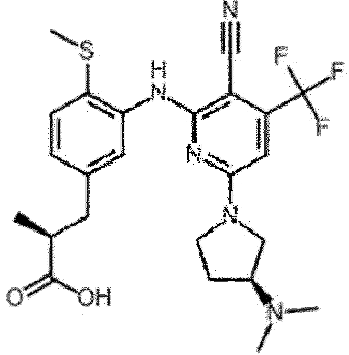 |

8. Sel d'un composé selon l'une quelconque des revendications 1 à 7, pour son utilisation comme médicament.

9. Médicament comprenant un composé ou un sel de celui-ci selon l'une quelconque des revendications 1 à 7.

10. Méthode de préparation du composé selon la revendication 1, comprenant la réaction de composés de chloropyridine de formule générale (II) 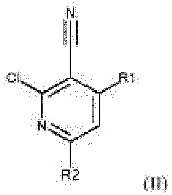
avec des anilines de formule générale (III) 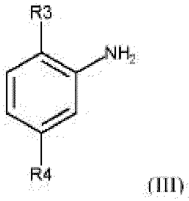
dans des conditions de couplage croisé en appliquant un catalyseur au palladium tel que. XPhos Pd G2 et une base telle que le carbonate de césium dans un solvant tel que le dioxane.

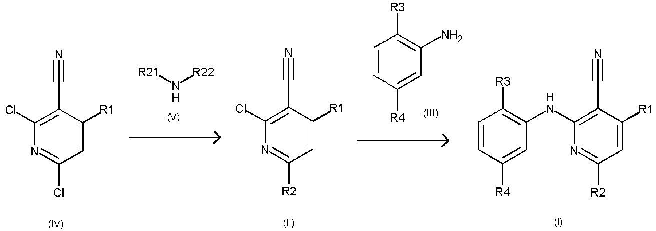

11. Méthode selon la revendication 10, **caractérisée en ce que** les composés de formule générale (II) sont préparés à partir de dichloropyridines de formule générale (IV) 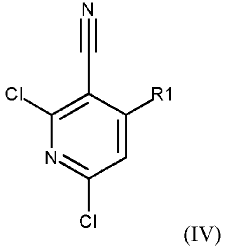 par réaction avec une amine primaire ou secondaire (V) appropriée en présence d'une base telle que la N,N-diisopropyléthylamine dans un solvant tel que l'éthanol.

12. Composition pharmaceutique contenant au moins un composé selon l'une quelconque des revendications 1 à 7 ou un sel pharmaceutiquement acceptable de celui-ci, conjointement avec un ou plusieurs supports pharmaceutiquement acceptables.

13. Composition pharmaceutique selon la revendication 12, comprenant une quantité thérapeutiquement efficace d'un composé selon l'une quelconque des revendications 1 à 7 dans la plage de 0,1 à 90 % en poids de la composition dans son ensemble, de préférence dans la plage de 0,5 à 50 % en poids de la composition dans son ensemble, ou un sel pharmaceutiquement acceptable de celle-ci,

14. Composé selon l'une ou plusieurs des revendications 1 à 7, ou sel pharmaceutiquement acceptable de celui-ci ou composition pharmaceutique selon la revendication 12, pour son utilisation dans le traitement ou la prévention de la stéatose hépatique non alcoolique (NAFLD), de la stéatohépatite non alcoolique (NASH) et du diabète de type II (T2DM).

15. Composé selon l'une quelconque des revendications 1 à 7, ou sel de celui-ci, pour son utilisation selon la revendication 14, dans lequel ledit composé est administré avant, après ou conjointement avec au moins une autre substance pharmaceutiquement active.
